(19) Europäisches Patentamt
European Patent Office
Office européen des brevets

(11) Veröffentlichungsnummer: **0 006 564**
**B1**

# (12) EUROPÄISCHE PATENTSCHRIFT

(45) Veröffentlichungstag der Patentschrift: **30.12.81**

(51) Int. Cl.³: **C 07 D 249/20,** C 08 K 5/34, C 09 D 5/00, C 09 D 7/12

(21) Anmeldenummer: **79102004.3**

(22) Anmeldetag: **18.06.79**

## (54) 2-(2-Hydroxy-3.5-disubstituiertes-phenyl)-2H-benzotriazol und damit stabilisierte Mischungen.

(30) Priorität: **26.06.78 US 918984**
**10.05.79 US 36914**
**16.05.79 US 38768**

(43) Veröffentlichungstag der Anmeldung: **09.01.80 Patentblatt 80/1**

(45) Bekanntmachung des Hinweises auf die Patenterteilung: **30.12.81 Patentblatt 81/52**

(84) Benannte Vertragsstaaten: **BE DE FR GB IT NL**

(56) Entgegenhaltungen:
**DE-A1-2 065 975**
**DE-A-2 130 322**
**DE-A1-2 647 452**
**DE-A1-2 755 340**
**US-A-3 072 585**
**US-A-3 189 615**
**US-A-3 983 132**

(73) Patentinhaber: **CIBA-GEIGY AG, Patentabteilung Postfach, CH-4002 Basel (CH)**

(72) Erfinder: **Dexter, Martin, 416 Cedar Drive West, Briarcliff Manor, N.Y. 10510 (US)**
Erfinder: **Winter, Roland A.E., 23 Banksville Road, Armonk New York 10504 (US)**

**EP 0 006 564 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

2-[2-Hydroxy-3,5-disubstituiertes-phenyl]-2H-benzotriazol und damit stabilisierte Mischung

Die vorliegende Erfindung betrifft 2-Aryl-2H-benzotriazolen, deren Verwendung als Lichtschutzmittel für organisches Material, sowie stabilisierte Mischungen.

Ferner betrifft die Erfindung ein Verfahren zur Herstellung von o-Nitroazobenzol Zwischenprodukten, die in das entsprechende 2-Aryl-2H-benzotriazol umgewandelt werden können.

UV-Absorber vom Typ der o-Hydroxyphenyl-2H-benzotriazolen geniessen seit langem ein erhebliches wirtschaftliches Interesse als Lichtstabilisatoren für organisches Material.

Solche Verbindungen, deren Herstellung und Verwendung dieser wertvollen 2-Aryl-2H-benzotriazolen sind z.B. aus den folgenden Patentschriften bekannt: US 3004896, US 3055896, US 3072585, US 3074910, US 3189615 und US 3230194. Diese Stabilisatoren zeigen jedoch eine schlechte Verträglichkeit in gewissen Substraten. Sie neigen stark zum Ausschwitzen, Sublimieren und/oder zum Verflüchtigen während der Verarbeitung zu Folien, Filmen, Fasern etc. bei hohen Temperaturen. Auf diese Weise geht ein unangemessen hoher Teil des Benzotriazolstabilisators verloren, insbesondere bei extrem dünnen Filmen und Überzügen. Dies gilt auch, wenn die Filme oder Überzüge während der Verwendung erhöhter Temperatur ausgesetzt sind.

Es sind Versuche unternommen worden, durch strukturelle Abwandlung des Benzotriazols, die Verträglichkeit zu verbessern und den Stabilisatorschwund zu reduzieren.

US-PS 3230194 beschreibt Benzotriazole, substituiert mit einer langkettigen Alkyl-Gruppe anstelle von Methyl-Gruppe, insbesondere 2-[2-Hydroxy-5-tert.-octylphenyl]-2H-benzotriazol, welche ausgezeichnete Verträglichkeit und stabilisierende Wirksamkeit zeigt in Polyäthylen.

US-PS 4127586 beschreibt andere Modifikationen von 2-Aryl-2H-benzotriazolen zur Verbesserung der Verarbeitungseigenschaften. So, die Verbindung 2-[2-Hydroxy-3-(1-phenyläthyl)-5-methylphenyl]-2H-benzotriazol zeigt bessere Verträglichkeit und geringeren Verlust durch Verflüchtigung während der Verarbeitung, als die bis dahin bekannten Benzotriazol-Verbindungen.

Aus JP Kokai 158588/75 sind ebenfalls Lichtstabilisatoren vom Typ Benzotriazol bekannt, wie z.B. 2-[2-Hydroxy-3-α,α-dimethylbenzyl-5-methylphenyl]-2H-benzotriazol.

Man ist weiterhin bestrebt, die Verarbeitungseigenschaften der Benzotriazolen während der Verarbeitung bei hohen Temperaturen zu verbessern.

Die erfindungsgemässen Verbindungen, wie z.B. 2-[Hydroxy-3,5-di-(α,α-dimethylbenzyl)phenyl]-2H-benzotriazol, zeigen überraschend geringen Verlust während der Hochtemperatur-Verarbeitung oder in deren Endverwendung, wo die stabilisierten Mischungen als Filme und Überzüge ständigen Witterungs- und Lichteinwirkungen ausgesetzt sind.

In US-PS 4041044 ist ein verbessertes Verfahren zur Herstellung der 2-Aryl-2H-benzotriazole beschrieben, nicht jedoch die erfindungsgemässen Verbindungen.

Die vorliegende Erfindung betrifft 2-Aryl-2H-benzotriazole als Lichtschutzmittel und mit 2-Aryl-2H-benzotriazolen stabilisiertes organisches Monomer und Polymer.

Die Erfindung betrifft insbesondere Verbindungen der Formel I

Verbindungen der Formel I

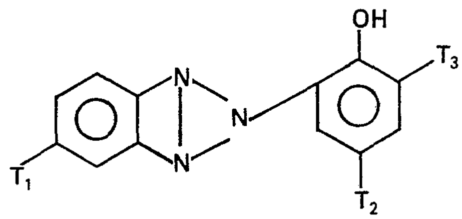

I

worin $T_1$ Wasserstoff oder Chlor ist, $T_2$ und $T_3$ unabhängig voneinander die Gruppe

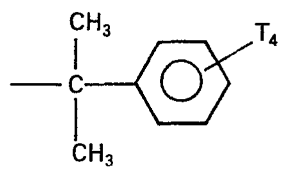

bedeuten, worin $T_4$ Wasserstoff oder niedriges Alkyl ist, wobei eines von $T_2$ und $T_3$ auch t-Octyl sein kann.

Ist $T_4$ ein niedriges Alkyl, so befindet es sich insbesondere in meta- oder para-Stellung, bevorzugt in der para-Stellung. Typische Alkyl-Reste sind solche mit 1-6 C-Atomen wie Methyl, Aethyl, i-Propyl, t-Butyl, t-Amyl und 3-Methylpentyl.

Bevorzugt sind Verbindungen worin $T_2$ und $T_3$ die gleiche Bedeutung haben und $T_4$ Wasserstoff oder p-Methyl ist.

Besonders bevorzugt ist $T_4$ Wasserstoff.

Besonders bevorzugt ist die Verbindung 2-[2-Hydroxy-3,5-di-(α,α-dimethyl-benzyl)-phenyl]-2H-benzotriazol.

Besonders bevorzugte Verbindungen sind 2-[2-Hydroxy-3-t-octyl-5-(α,α-dimethyl-benzyl)-phenyl]-2-H-benzotriazol und 2-[2-Hydroxy-3-(α,α-dimethyl-benzyl)-5-t-octylphenyl]-2H-benzotriazol.

Die erfindungsgemässen Verbindungen werden wie folgt hergestellt:

Stufe I:

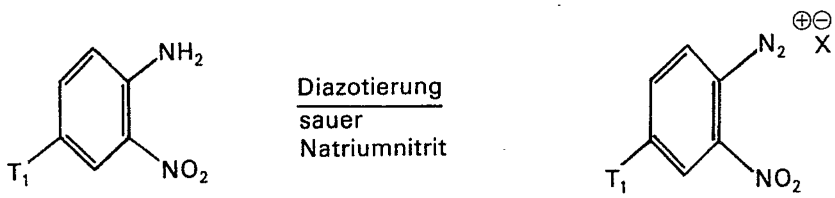


III.

IV.

X ist ein Anion wie z. B. Chlor oder Sulfat.

IV. +

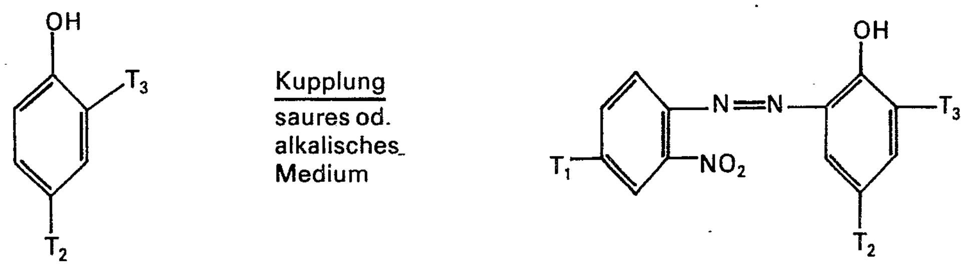


V.

VI.

Stufe II:

VI.

reductive
Cyclisierung

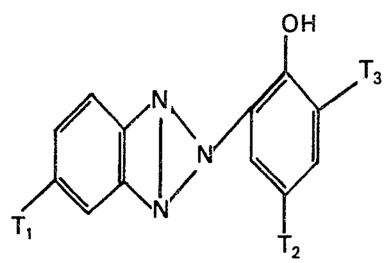

I.

$T_1$, $T_2$ und $T_3$ haben die zuvor genannte Bedeutung.

1. Stufe – Die Kupplung der Diazonium-Verbindung kann in saurem oder alkalischem Milieu erfolgen. Falls das gekuppelte Phenol eine Aralkyl-Gruppe in ortho-Stellung zur Hydroxyl-Gruppe trägt und die Reaktion in saurem Milieu durchgeführt wird, so ist die Ausbeute an o-Nitroazobenzol VI. 50% unter der Theorie.

Überraschenderweise erhält man vom Zwischenprodukt VI. über 80% der Theorie, wenn man die Kupplungsreaktion in stark alkalischem Milieu (pH>11) ausführt.

2. Stufe – Die reduktive Cyclisierung von VI. zu 2-Aryl-2H-benzotriazol durch einer der bekannten Reduktionsmethoden, wie z.B. Zink und NaOH, Hydrazin oder katalytische Hydrierung mit einem Edelmetall-Katalysator, erfolgen. Die Verwendung dieser Mittel führt zu guten Benzotriazol-Ausbeuten.

Ferner betrifft die Erfindung ein Verfahren zur Herstellung von o-Nitroazobenzol Zwischenprodukten, die in das entsprechende 2-Aryl-2H-benzotriazol umgewandelt werden können.

Das erfindungsgemässe Verfahren betrifft die Kupplung eines substituierten Phenols mit diazotiertem Anilin in einem stark alkalischen Alkanol. Ein Minimum an Wasser in der Reaktionsmischung ist vorteilhaft für die Löslichkeit des substituierten Phenols.

Das erfindungsgemässe Verfahren unterscheidet sich vom Stand der Technik in der Verwendung eines Überschusses an Alkalihydroxid, der weit über die stöchiometrische Menge hinausgeht, welche zum Neutralisieren des entstandenen Diazoniumsalzes und zur Salzbildung des Phenols notwendig ist. Demgemäss erfolgt die Kupplung in Gegenwart überschüssigen Hydroxyl- Ionen und bei einem pH-Wert von über 11.

Der Stand der Technik lehrt das Vermeiden von stark alkalischen Kupplungsprozessen. Daher ist der Erfolg, mit dem der gegenwärtige Kupplungsprozess durchgeführt wurde besonders überraschend.

Die Kupplung der Phenole mit Diazoniumsalz unter schwach alkalischen Bedingungen ist eine bekannte Methode für die Herstellung von aromatischen Azo-Derivaten. In «Basic Principles of Organic Chemistry» von J. D. Roberts und M. C. Caserio, W. A. Benjamin, Inc. New York (1965), S. 893-895 wird betont, dass der Kupplungsprozess bei etwa pH 10 optimal verläuft, bei höheren pH-Werten aber praktisch aufhört.

Ferner ist es bekannt, dass o-Nitrobenzoldiazoniumsalz-Lösungen in Gegenwart von Alkohol und Alkalimetallhydroxid instabil sind und unter $N_2$-Entwicklung sich schnell zersetzen. Aus diesem Grund lehrt der Stand der Technik Kupplungsreaktionen in überschüssigem Alkalihydroxid zu vermeiden. Siehe Fierz-David et al, «Fundamental Processes of Dye Chemistry», Interscience, New York (1949), S. 239-241, 252-253.

H. Zollinger «Azo and Diazo Chemistry», Interscience, New York (1961), S. 249-250 berichtet, dass sich bei stark alkalischer Kupplung das Gleichgewicht verschiebt von der Seite der Diazonium-lonen.

SU-PS 360 357 beschreibt die Kupplung eines Natriumphenolates in schwach alkalischer Lösung mit überschüssigem sauren Diazoniumsalz, so dass der pH rapid unter den Wert 11 fällt während der Reaktion, ganz im Gegensatz zu den erfindungsgemässen Reaktionsbedingungen.

Im Gegensatz zu den Lehren des Standes der Technik o-Nitroazobenzol wird in hoher Ausbeute und in grosser Reinheit gewonnen, wenn die Reaktion in stark alkalischem Milieu und in Gegenwart eines niedrigen Alkanols als Lösungsmittel durchgeführt wird.

Die Herstellung von o-Nitroazobenzol Zwischenprodukt, welche zur Herstellung von 2-Aryl-2H-benzotriazol Lichtstabilisatoren verwendet wird, ist Gegenstand einer Teilanmeldung.

Die erfindungsgemässen Verbindungen sind wirksame Lichtstabilisatoren für eine grosse Anzahl organischer Polymere. Solche Polymere sind:

1. Polymere, welche von Mono- oder Diolefinen stammen, z. B. gegebenenfalls vernetztes Polyäthylen, Polypropylen, Polyisobutylen, Polymethylbuten-1, Polymethylpenten-1-, Polyisopren und Polybutadien.

2. Gemische der Homopolymere von 1), z. B. Gemische von Polypropylen und Polyethylen, Polypropylen und Polybuten-1, Polypropylen und Polyisobutylen.

3. Copolymere aus den Monomeren für die Homopolymeren unter 1), z. B. Aethylen/Propylen-Copolymer, Propylen/Butene-1-Copolymer, Propylenmer/Isobutylen-Copolymer, Aethylen/Buten-1-Copolymer sowie Terpolymere von Aethylen und Propylen mit einem Dien, z. B. Hexadin, Dicyclopentadien oder Aethyliden-Norbornen, und Copolymere von α-Olefine, z. B. Aethylen mit Acryl- oder Methacrylsäure.

4. Polystyrol.

5. Copolymere von Styrol und von α-Methylstyrol, z. B. Styrol/Butadine-Copolymer, Styrol/Acrylnitril-Copolymer, Styrol/Acrylnitril/Methacrylate-Copolymer, Styrol/Acrylnitril-Copolymer modifiziert mit Acrylester-Polymer z. B. Styrol/Butadine/Styrol-Block-Copolymer.

6. Graft-copolymere von Styrol, z. B. das Graft-polymer von Styrol zu Polybutadin, das Graft-polymer von Styrol mit Acrylnitril zu Polybutadin sowie ihre Gemische mit den Copolymeren von 5), bekannt als Acrylnitril/Butadin/Styrol oder ABS.

7. Halogenhaltige Vinyl-polymere, z. B. Polyvinylchlorid, Polyvinylidenchlorid, Polyvinylfluorid, Polychloropren, chloriniertes Gummi, Vinylchlorid/Vinylidenchlorid-copolymer, Vinylchlorid/Vinylacetat-copolymer, Vinylidenchlorid/Vinylacetat-copolymer.

8. Polymere, welche von α,β-ungesättigten Säuren stammen und ihre Derivate, Polyacrylate und Polymethacrylate, Polyacrylamide und Polyacrylnitril. Die erfindungsgemässen Verbindungen werden vorteilhafterweise in hitzehärtbaren Acrylharz-Lacken verwendet, welche aus einer Acrylsäure und einem Melanin-Formaldehydharz bestehen.

9. Polymere, hergestellt aus ungesättigten Alkoholen und Aminen und von deren Acyl-derivativen oder Acetalen, z. B. Polyvinylalkohol, Polyvinylacetat, Polyvinylstearat, Polyvinylbenzoat, Polyvinylmaleat, Polyvinylbutyral, Polyallylphtalat, Polyallylmelamin und ihre Copolymere mit anderen Vinyl-Verbindungen z. B. Äthylen/Vinylacetat-copolymer.

10. Homopolymere und Copolymere hergestellt aus Epoxiden z. B. Polyäthylenoxid oder die Polymere der bis-Glycidyläther.

11. Polyacetate, z. B. Polyoxymethylen, sowie Polyoxymethylene, welche Äthylenoxid als Comonomer enthalten.

12. Polyalkylenoxide z. B. Polyoxyäthylen, Polypropylenoxid oder Polyisobutylenoxid.

13. Polyphenylenoxide.

14. Polyurethane und Polyharnstoffe, wie in Urethanüberzügen.

15. Polycarbonate.

16. Polysulfone.

17. Polyamide und Copolyamide, hergestellt aus Diaminen und Dicarboxylsäuren und/oder aus Aminocarboxylsäuren oder deren Lactamen, z. B. Polyamid 6, Polyamid 6/6, Polyamid 6/10, Polyamid 11, Polyamid 12, Poly-m-phenylen-isophtalamid.

18. Polyester aus Dicarboxylsäuren und Dialkoholen und/oder aus Hydroxycarboxylsäuren oder deren Lactonen, z. B. Polyäthylenglycolterephtalat, Poly-1,4-dimethylol-cyclohexanterephtalat

19. Vernetzte Polymere aus Aldehyden einerseits und aus Phenolen, Harnstoffen und Melamin, anderseits z. B. Phenol/Formaldehyd, Harnstoff/Formaldehyd und Melamin/Formaldehyd-Harz.

20. Alkyd-Harze z. B. Glycerol/Phtalsäure-Harze und deren Gemische mit Melamin/Formaldehyd-Harzen.

21. Ungesättigte Polyester-Harze aus den Copolyestern der gesättigten und ungesättigten Dicarboxylsäuren mit mehrwertigen Alkoholen sowie aus Vinyl-Verbindungen als Vernetzungsmittel und deren halogenhaltigen, flammenresistenten Modifikationen.

22. Natürliche Polymere, z. B. Cellulose, Gummi sowie deren chemisch modifizierten Homolog-Derivative, z. B. Celluloseacetate, Cellulosepropionate und Cellulosebutyrate und die Celluloseäther, z. B. Methylcellulose.

Die erfindungsgemässen Verbindungen sind nicht nur wirksame Lichtschutzmittel, wie die 2-Aryl-2H-benzotriazole im allgemeinen, sondern

auch besonders wertvoll zum Stabilisieren von polymeren Substraten, welche bei hohen Temperaturen verarbeitet werden müssen, dank ihres überraschend geringen Verlustes durch Verflüchtigung bei hoher Temperatur.

Die erfindungsgemässen Verbindungen sind daher besonders geeignet zum Stabilisieren von Polymeren, wie z. B. Polyäthylenterephtalat, Polybutylenterephtalat oder deren Copolymere, Polycarbonate, z. B. aus Bisphenol A und Phosgen oder deren Copolymere, Polysulfone, Polyamide, z. B. Nylon-6, Nylon-6,6, Nylon-6/10 etc. sowie Copolyamide, duroplastische Acrylharze, thermoplastische Acrylharze, Polyolefine, z. B. Polyäthylen, Polypropylen, Copolyolefine etc. und andere Polymere, welche Hochtemperatur-Verarbeitung und -Produktion verlangen.

Die Stabilisatoren der Formeln I. und II. werden nach den herkömmlichen Methoden in das Polymer eingearbeitet, und zwar in jeder beliebigen Phase während der Herstellung von geformten Produkten. Sie können zum Beispiel als Pulver, Suspension oder Emulsion zum Polymer gemischt werden, welches als Pulver, Suspension oder Emulsion vorliegen kann.

Die stabilisierten polymeren Mischungen der Erfindung können gegebenenfalls 0,1 bis 5 Gew.%, vorzugsweise 0,5 bis 3 Gew.% der üblichen Zusatzstoffe verwendet werden, insbesondere Antioxydantien, Lichtstabilisatoren, oder deren Gemische. Beispiele für solche Zusatzstoffe:

Antioxydantien, UV-Absorber und Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, des weiteren Nickelverbindungen, sterisch gehinderte Amine, Oxalsäurediamide, Metalldesaktivatoren, Phosphite, peroxidzerstörende Verbindungen Polyamidstabilisatoren, basische Co-Stabilisatoren, Nukleierungsmittel oder sonstige Zusätze wie z. B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Gewisse hydrophobische, nicht-diffundierende Hydroxyphenyl-benzotriazole sind bekannt als UV-Absorber in photographischer Gelatine. (US-PS 3253921 und US 4042394). Die erfindungsgemässen Verbindungen sind durch ihre geringe Flüchtigkeit, durch ihre guten Absorbtionseigenschaften im UV-Bereich und durch ihre photographische Inaktivität besonders gut geeignet zum Stabilisieren von Farbbildern.

Von besonderem Interesse sind die duroplastischen und thermoplastischen Acrylharze, welche für Autolackierungen verwendet werden. Diese Stoffe sind im Encyclopedia of Polymer Science and Technology, Interscience Publishers, New York, Band 1 (1964), auf Seiten 273-276 und Band 13 (1970), auf Seiten 530-532 beschrieben, ferner in «Understanding Paint» von W.R. Fuller, in American Paint Journal Co., St. Louis, 1965, Seite 124-135.

Acrylharzlacke, welche gemäss der Erfindung gegen die Einwirkung von Licht, Sauerstoff und Feuchtigkeit stabilisiert werden, sind die üblichen Einbrennlacke, wie z. B. in H. Kittel's «Lehrbuch der Lacke und Beschichtungen», Band 1, Teil 2, Seite 735 und 742 (Berlin, 1972), und in H. Wagner, H. F. Sarx, «Lackkunstharze», Seiten 229-235 beschrieben.

Von besonderem Interesse ist die Stabilisierung der mit den erfindungsgemässen Verbindungen, auf der Basis von heissvernetzbarem Acrylharz und Styrol. Aus diesen Harzen hergestellter Metalleffektlack ist trotz der ausgezeichneten physikalischen und chemischen Eigenschaften ungeeignet, da das vorhandene Styrol zur Rissbildung führt. Andere Lacke und Beschichtungen sind auf der Basis von Acryl/Melaminharz und Alkyd/Acryl/Melaminharz ebenfalls interessant.

Um die Metalleffektwirkung zu erzielen, verwendet man die üblichen Aluminium-Pigmente in 1-10 Gew.% bezogen auf das lösungsmittelfreie Bindemittel (Lackharz). Das Auftragen der stabilisierten Lacke kann nach den herkömmlichen Einschicht- oder Zweischicht-Verfahren geschehen. Im letzteren Falle, der Pigment enthaltende Vorlegelack wird zuerst aufgetragen und nachher mit Klarlack überzogen.

Weitere Zusatzstoffe, die im Lack enthalten sein können, sind andere übliche Lichtschutzmittel, phenolische Antioxydantien, Pigmente, Farbstoffe, Metalldesaktivatoren etc.

Im allgemeinen betragen die erfindungsgemäss verwendeten Stabilisatoren 0,1 bis 5 Gew.%, vorzugsweise 0,5 bis 3 Gew.%, bezogen auf das Lackharz.

Die Kombination von sterisch gehinderten Aminen und den erfindungsgemässen Stabilisatoren ermöglicht sowohl eine ausgezeichnete Glanzerhaltung als auch eine Beständigkeit gegen Abblätterung der metallisierten Einbrennlakke auf Acrylharzbasis für Automobil-Decklackierung.

Die sterisch gehinderten Amine sind in den Acrylharzlacken als Lichtstabilisatoren wirksam und verantwortlich für deren Glanzerhaltung bei Bewitterung. Das UV-Licht kann jedoch die Deckschicht, falls kein UV-Absorber vorhanden, ungehindert passieren und in der unteren Epoxyesterschicht zu Schäden führen. Durch die Zugabe der erfindungsgemässen Benzotriazol-UV-Absorbern wird dies erfolgreich verhindert.

Auf diese Weise bietet die Kombination von sterisch gehinderten Aminen mit Benzotriazolen sowohl Glanzerhaltung als auch Abblätterungsbeständigkeit in metallisierten Decklacken auf der Basis von Acrylharzen.

Die eingesetzten Amine sind in 0,1 bis 5 Gew.-%, vorzugsweise in 0,5 bis 2 Gew.-%, insbesondere in 0,5 bis 1 Gew.% wirksam, bezogen auf das Acrylharz.

Die erfindungsgemässen Benzotriazole sind in 0,1 bis 5 Gew.%, vorzugsweise in 0,5 bis 2 Gew.%, insbesondere bei 0,5 bis 1 Gew.% wirksam, bezogen auf das Acrylharz.

Die Amin-Lichtstabilisatoren, die in dieser Erfindung zusätzlich verwendet werden entsprechen der Formel XI.

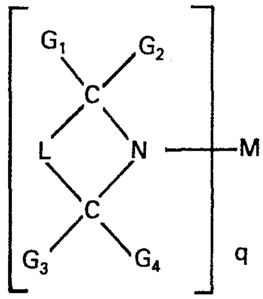

oder

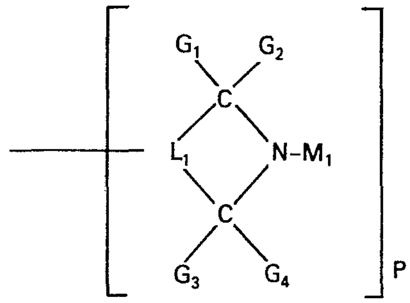

XI

worin q 1 oder 2 ist, p 2 bis 14 ist, $G_1$,$G_2$, $G_3$ und $G_4$ unabhängig voneinander Alkyl bedeuten, oder $G_1$ und $G_3$ zusammen ein Alkylen bilden, oder jeder Carboalkoxy oder Carbophenäthoxy ist, oder $G_1$ und $G_2$ oder $G_3$ und $G_4$, unabhängig voneinander, zusammen ein Alkylen oder Azaalkylen bilden; falls q 1 ist, M Wasserstoff, Hydroxy, Oxy 1, gegebenenfalls substituiertes Alkyl, Alkenyl, Alkynyl, Aralkyl, Alkanoyl, Alkenoyl, Benzoyl, Glycidyl oder $-CH_2CHOHZ$ ist, wo Z Wasserstoff, Methyl oder Phenyl bedeutet, falls q 2 ist, M Alkylen, Alkenylen, Alkynylen, Arylendialkylen, die Gruppe $-(CH)_2)_2OOCR_{18}COO(CH)_2)_2-$, oder die Gruppe $-CH_2OOOCR_{19}COOCH_2-$ ist, worin $R_{18}$ Alkylen und $R_{19}$ Alkylen, Xylylen oder Cyclohexylen sind, $M_1$ die gleiche Bedeutung wie M hat, wo q 1 ist,

L ein zweiwertiger organischer Rest ist, welcher den N-haltigen Ring zu einem 5- bis 7-Ring ergänzt, oder zwei einwertige Radikale bedeutet, und

$L_1$ ein zweiwertiger organischer Rest, welcher den N-haltigen Ring zu einem 5- bis 7-Ring ergänzt und welcher zusätzlich durch eine Verbindungsgruppe mit anderen Amingruppierungen verbunden sein kann.

Als Amine kommen die Verbindungen der folgenden Formel in Frage:

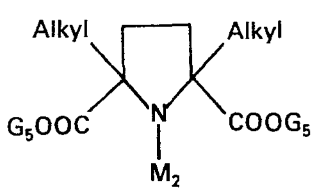

XII

worin $G_5$ Wasserstoff, Alkyl oder Phenäthyl ist und $M_2$ Wasserstoff, hydroxy, Oxyl, Alkyl, 2-Methoxyäthyl, Alkenyl oder Propagyl bedeutet.

Bevorzugt werden 2,2,6,6-Tetraalkylpiperidinverbindungen verwendet, welche vorzugsweise eine Gruppe der Formel XIII

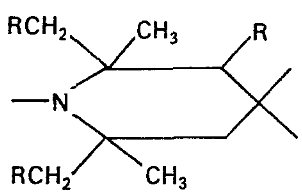

XIII

enthalten, worin R Wasserstoff oder Methyl bedeutet.

Zu den erfindungsgemäss zu verwendenden Lichtschutzmitteln gehören insbesondere folgende Verbindungsklassen:

a) Lichtschutzmittel der Formel XIV

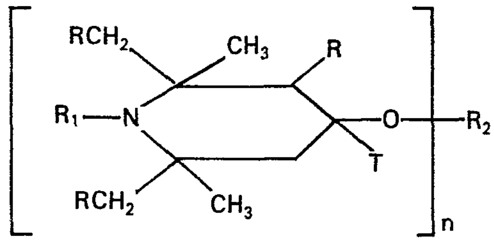

XIV

worin n die Zahlen 1-4, vorzugsweise 1 oder 2 bedeutet, T Wasserstoff oder $-CONH_2$ ist, R die für Formel XIII angegebene Bedeutung hat, $R_1$ Wasserstoff, Oxyl, $C_1$-$C_{18}$ Alkyl, $C_3$-$C_8$ Alkenyl, $C_3$-$C_8$ Alkinyl, $C_7$-$C_{12}$ Aralkyl, $C_1$-$C_8$ Alkenoyl, Glycidyl, eine Gruppe $-CH_2CH(OH)-Z$, worin Z Wasserstoff, Methyl, Äthyl oder Phenyl ist, wobei $R_1$ vorzugsweise Wasserstoff, $C_1$-$C_{12}$ Alkyl, Allyl, Benzyl, Acetyl, Acryloyl ist und $R_2$, wenn n=1, Wasserstoff, gegebenenfalls durch ein oder mehrere Sauerstoffatome unterbrochenes $C_1$-$C_{18}$ Alkyl, Cyanäthyl, Benzyl, Glycidyl, einen einwertigen Rest einer aliphatischen, cycloaliphatischen, araliphatischen, aromatischen oder heterocyclischen Carbonsäure, Carbaminsäure oder Phosphor enthaltenden Säure oder einen einwertigen Silylrest, vorzugsweise einen Rest einer aliphatischen Carbonsäure mit 2-18 C-Atomen, einer cycloaliphatischen Carbonsäure mit 5-12 C-Atomen oder einer aromatischen Carbonsäure mit z-15 C-Atomen, wenn n = 2, $C_1$-$C_{12}$ Alkylen, $C_4$-$C_{12}$ Alkenylen, Xylylen, einen zweiwertigen Rest einer aliphatischen, cycloaliphatischen, araliphatischen, aromatischen oder heterocyclischen Dicarbonsäure, Dicarbaminsäure oder Phosphor enthaltenden Säure oder einen zweiwertigen Silylrest, vorzugsweise einen Rest einer aliphatischen Dicarbonsäure mit 2-36 C-Atomen, einer cycloaliphatischen oder aromatischen Dicarbonsäure mit 8-10 C-Atomen, einer aliphatischen, cycloaliphatischen oder aromatischen Dicarbaminsäure mit 8-10 C-Atomen, wenn n = 3, einen dreiwertigen Rest einer aliphatischen, cycloaliphatischen, aromatischen oder heterocyclischen Tricarbonsäure, einer aromatischen Tricarbaminsäure oder einer Phosphor enthaltenden Säure oder einen dreiwertigen Silylrest, wenn n = 4,

einen vierwertigen Rest einer aliphatischen, cycloaliphatischen oder aromatischen Tetracarbonsäure bedeutet.

Bedeuten etwaige Substituenten $C_1$–$C_{12}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl oder n-Dodecyl dar.

In der Bedeutung von $C_1$–$C_{18}$ Alkyl können $R_1$ oder $R_2$ z. B. die oben angeführten Gruppen und dazu noch beispielsweise n-Tridecyl, n-Tetradecyl, n-Hexadecyl oder n-Octadecyl darstellen.

Wenn $R_1$ $C_2$–$C_8$ Alkenyl bedeutet, so kann es sich z. B. um 1-Propenyl, Allyl, Methallyl, 2-Butenyl, 2-Pentenyl, 2-Hexenyl, 2-Octenyl, 4-tert.-Butyl-2-butenyl handeln.

$R_1$ ist als $C_3$–$C_8$ Alkinyl bevorzugt Propargyl.

Als $C_7$–$C_{12}$ Aralkyl ist $R_1$ insbesondere Phenäthyl oder vor allem Benzyl.

$R_1$ ist als $C_1$–$C_8$ Alkanoyl beispielsweise Formyl, Propionyl, Butyryl, Octanoyl aber bevorzugt Acetyl und als $C_3$–$C_5$ Alkenoyl insbesondere Acryloyl.

Bedeutet $R_2$ einen einwertigen Rest einer Carbonsäure, so stellt es beispielsweise einen Essigsäure-, Stearinsäure-, Salicylsäure-, Methacrylsäure-, Maleinsäure-, Benzoe- oder β-(3,5-Di-tert.-butyl-4-hydroxyphenyl)-propionsäurerest dar.

Bedeutet $R_2$ einen zweiwertigen Rest einer Dicarbonsäure, so stellt es beispielsweise einen Adipinsäure-, Suberinsäure-, Sebacinsäure-, Phtalalsäure-, Dibutylmalonsäure-, Dibenzylmalonsäure-, Butyl-(3,5-di-tert.-butyl-4-hydroxybenzyl)-malonsäure- oder Bicyclohepten-dicarbonsäurerest dar.

Stellt $R_2$ einen dreiwertigen Rest einer Tricarbonsäure dar, so bedeutet es z. B. einen Trimellithsäure- oder einen Nitrilotriessigsäurerest.

$R_2$ kann auch Reste von Di- und Tricarbonsäuren bedeuten, wie sie technisch durch Oligomerisation höherer ungesättigter Fettsäuren oder durch Diels-Alder Addition von Crylsäure an Linolsäure hergestellt werden. Ferner kann $R_2$ auch Reste bedeuten, die durch Umsetzung von Mono-, Di- und Polyepoxiden wie z. B. Biphenol A-diglycidyläther, Butandialdiglycidäther, Trisglycidyl-isocyanurat, 1,3-Diglycidyl-4,4-dimethylhydantoin mit 4-Hydroxy-1,2,2,6,6-pentamethylpiperidin entstehen.

Stellt $R_2$ einen vierwertigen Rest einer Tetracarbonsäure dar, so bedeutet es z.B. einen Pyromellithsäurerest.

Bedeutet $R_2$ einen zweiwertigen Rest einer Dicarbaminsäure, so stellt es beispielsweise einen Hexamethylendicarbaminsäure- oder einen 2,4-Toluylen-dicarbaminsäurerest dar.

Beispiele für Polyalkylpiperidin-Lichtschutzmittel dieser Klase sind folgende Verbindungen:

1) 4-Hydroxy-2,2,6,6-tetramethylpiperidin
2) 1-Allyl-4-hydroxy-2,2,6,6-tetramethylpiperidin
3) 1-Benzyl-4-hydroxy-2,2,6,6-tetramethylpiperidin
4) 1-(4-tert.-Butyl-2-butenyl)-4-hydroxy-2,2,6,6-tetramethylpiperidin
5) 4-Stearoyloxy-2,2,6,6-tetramethylpiperidin
6) 1-Äthyl-4-salicyloyloxy-2,2,6,6-tetramethylpiperidin
7) 4-Methacryloyloxy-1,2,2,6,6-pentamethylpiperidin
8) 1,2,2,6,6-Pentamethylpiperidin-4-yl-β-(3,5-di-tert.-butyl-4-hydroxyphenyl)-propionat
9) 1-Benzyl-2,2,6,6-tetramethyl-4-piperidinylmaleinat
10) Bis-(2,2,6,6-tetramethylpiperidin-4-yl)-adipat
11) Bis-(2,2,6,6-tetramethylpiperidin-4-yl)-sebacat
12) Bis-(1,2,3,6-tetramethyl-2,6-diäthyl-piperidin-4-yl)-sebacat
13) Bis-(1-allyl-2,2,6,6-tetramethyl-piperidin-4-yl)-phtalat
14) 1-Propargyl-4-β-cyanoäthyloxy-2,2,6,6-tetramethylpiperidin
15) 1-Acetyl-2,2,6,6-tetramethylpiperidin-4-yl-acetat
16) Trimellithsäure-tri-(2,2,6,6-tetramethylpiperidin-4-yl)-ester
17) 1-Acryloyl-4-benzyloxy-2,2,6,6-tetramethylpiperidin
18) Dibutyl-malonsäure-di-(1,2,2,6,6-pentamethylpiperidin-4-yl)-ester
19) Butyl-(3,5-di-tert.-butyl-4-hydroxybenzyl)-malonsäure-di-(1,2,2,6,6-pentamethylpiperidin-4-yl)-ester
20) Dibenzyl-malonsäure-di-(1,2,2,6,6-pentamethylpiperidin-4-yl)-ester
21) Dibenzyl-malonsäure-di-(1,2,3,6-tetramethyl-2,6-diäthyl-piperidin-4-yl)ester
22) Hexan-1′, 6′-bis-(4-carbamoyloxy-1-n-butyl-2,2,6,6-tetramethyl-piperidin)
23) Toluol-2′,4′-bis-(4-carbamoyloxy-1-n-propyl-2,2,6,6-tetramethyl-piperidin)
24) Dimethyl-bis-(2,2,6,6-tetramethylpiperidin-4-oxy)-silan
25) Phenyl-tris-(2,2,6,6-tetramethylpiperidin-4-oxy)-silan
26) Tris-(1-propyl-1-2,2,6,6-tetramethylpiperidin-4-yl)-phosphit
27) Tris-(1-propyl-2,2,6,6-tetramethylpiperidin-4-yl)-phosphat
28) Phenyl-[bis-(1,2,2,6,6-pentamethylpiperidin-4-yl)]-phosphonat
29) Bis-(1,2,2,6,6-pentamethylpiperidin-4-yl)-sebacat
30) Bis-(1-benzyl-2,2,6,6-tetramethylpiperidin-4-yl)-sebacat
31) Bis-(1-allyl-2,2,6,6-tetramethylpiperidin-4-yl)-sebacat
31a) 2,2,6,6-Tetramethyl-4-hydroxy-4-carbamoylpiperidin
32) 4-Benzoyloxy-2,2,6,6-tetramethylpiperidin
33) 4-Acryloxy-2,2,6,6-tetramethylpiperidin
34) 4-(p-Chlorbenzoyloxy)-1,2,2,6,6-pentamethylpiperidin
35) 4-Lauroyloxy-1,2,2,6,6-pentamethylpiperidin
36) 1-Allyl-2,2,6,6-tetramethyl-4-piperidinyl-salicylat
37) 1-(2-Benzoyloxyäthyl)-2,2,6,6-tetramethyl-4-piperidinylbenzoat

38) Bis-(1,2,2,6,6-pentamethyl-4-piperidinyl)-isophtalat
39) Bis-(1,2,2,6,6-pentamethyl-4-piperidinyl)-adipat
40) Bis-(1-acetyl-2,2,6,6-tetramethyl-4-piperidinyl)-sebacat
41) Bis-(1-hydroxyäthyl-2,2,6,6-tetramethyl-4-piperidinyl)-succinat
42) Tris-(2,2,6,6-tetramethyl-4-piperidinyl)-nitrilotriacetat
43) Tris-(1-butyl-2,2,6,6-tetramethyl-4-piperidinyl)-trimellithat
44) Tris-(1-butyl-2,2,6,6-tetramethyl-4-piperidinyl)-phosphat
45) Diphenyl-bis-(2,2,6,6-tetramethylpiperidin-4-oxy)-silanester
46) 0,0'-Di(2,2,6,6-tetramethyl-4-piperidinyl)-tolylen-1,4-di-carbamat
47) 1-Methylcarbamoyl1-2,3,6-trimethyl-2,6-diäthyl-4-piperidinyl-methylcarbamat.

b) Lichtschutzmittel der Formel XV

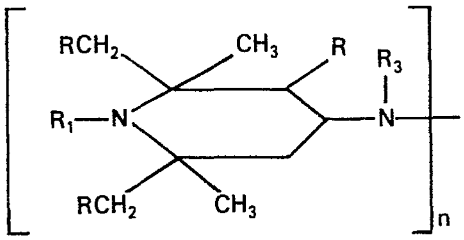
XV

worin n die Zahlen 1 oder 2 bedeutet, R die für Formel XIII angegebene Bedeutung hat, $R_1$ die unter a) angegebene Bedeutung hat, $R_3$ Wasserstoff, $C_1$-$C_{12}$ Alkyl, $C_5$-$C_7$ Cycloalkyl, $C_7$-$C_8$ Aralkyl, $C_2$-$C_{18}$ Alkanoyl, $C_3$-$C_5$ Alkenoyl, Benzoyl ist und $R_4$ wenn n = 1, Wasserstoff, $C_1$-$C_{18}$ Alkyl, $C_5$-$C_7$ Cycloalkyl, gegebenenfalls mit einer Cyano-, Carbonyl oder Carbamidgruppe substituiertes $C_2$-$C_8$ Alkenyl, Glycidyl, eine Gruppe der Formel $-CH_2-CH(OH)-Z$ oder der Formel -CONH-Z ist, worin Z Wasserstoff, Methyl oder Phenyl bedeutet; wenn n = 2, $C_2$-$C_{12}$ Alkylen, $C_6$-$C_{12}$ Arylen, Xylylen, eine $-CH_2-CH(OH)-CH_2$-Gruppe oder eine Gruppe $-CH_2-CH(OH)-CH_2-O-X-O-CH_2-$ $-CH(OH)-CH_2-$ bedeutet, worin X $C_2$-$C_{10}$ Alkylen, $C_6$-$C_{15}$ Arylen, $C_6$-$C_{12}$ Cycloalkylen ist, oder, vorausgesetzt, dass $R_3$ nicht Alkanoyl, Alenoyl oder Benzoyl bedeutet, $R_4$ auch einen zweiwertigen Rest einer aliphatischen, cycloaliphatischen oder aromatischen Dicarbonsäure oder Dicarbaminsäure oder auch die Gruppe -CO-bedeuten kann, oder $R_3$ und $R_4$ zusammen, wenn n = 1, den Imidrest einer aliphatischen, cycloaliphatischen oder aromatischen 1,2- oder 1,3-Dicarbonsäure sein können.

Stellen etwaige Substituenten $C_1$-$C_{18}$ Alkyl dar, so haben sie die bereits unter a) angegebene Bedeutung.

Bedeuten etwaige Substituenten $C_5$-$C_7$ Cycloalkyl, so stellen sie insbesondere Cyclohexyl dar.

Als $C_7$-$C_8$ Aralkyl ist $R_3$ insbesondere Phenäthyl oder vor allem Benzyl.

$R_3$ ist als $C_2$-$C_{18}$ Alkanoyl beispielsweise Propionyl, Butyryl, Octanoyl, Dodecanoyl, Hexadecanoyl, Octadecanoyl aber bevorzugt Acetyl und als $C_3$-$C_5$ Alkenoyl insbesondere Acryloyl.

Bedeutet $R_4$ ein gegebenenfalls mit einer Cyano-, Carbonyl- oder Carbamidgruppe substituiertes $C_2$-$C_8$ Alkenyl, dann handelt es sich z. B. um 1-Propenyl, Allyl, Methallyl, 2-Butenyl, 2-Phentenyl, 2-Hexenyl, 2-Octenyl, 2,2-Dicyanovinyl, 1-Methyl-2-cyano-2-methoxycarbonyl-vinyl, 2,2-Diacetylaminovinyl.

Stellen etwaige Substituenten $C_2$-$C_{12}$ Alkylen dar, so handelt es sich z.B. um Äthylen, Propylen, 2,2-Dimethylpropylen, Tetramethylen, Hexamethylen, Octamethylen, Decamethylen oder Dodecamethylen.

Bedeuten etwaige Substituenten $C_6$-$C_{15}$ Arylen, so stellen sie z.B. o-, m- oder p-Phenylen, 1,4-Naphtylen oder 4,4'-Diphenylen dar.

Als $C_6$-$C_{12}$ Cycloalkylen ist X insbesondere Cyclohexylen.

Beispiele für Polyalkylpiperidin-Lichtschutzmittel dieser Klasse sind folgende Verbindungen:

48) N,N'-Bis-(2,2,6,6-tetramethylpiperidin-4-yl)-hexamethylen-1,6-diamin
49) N,N'-Bis-(2,2,6,6-tetramethylpiperidin-4-yl)-hexamethylen-1,6-diacetamid
50) 4-Acetylamino-1,2,2,6,6-pentamethylpiperidin
51) 1-Benzyl-2,2,6,6-tetramethyl-4-diäthanolamino-piperidin
52) 4-Acrylamido-1,2,2,6,6-pentamethylpiperidin
53) 1-Acetyl-4-(N-cyclohexylacetamido)-2,2,6,6-tetramethylpiperidin
54) 4-Benzylamino-2,2,6,6-tetramethylpiperidin
55) N,N'-Bis-(2,2,6,6-tetramethylpiperidin-4-yl)-N,N'-dibutyl-adipamid
56) N,N'-Bis-(2,2,6,6-tetramethylpiperidin-4-yl)-N,N'-dicyclohexyl-(2-hydroxypropylen)
57) N,N'-Bis-(2,2,6,6-tetramethylpiperidin-4-yl)-p-xylylen-diamin
58) N,N'-Bis-(1,2,2,6,6-pentamethyl-4-piperidinyl)-1,6-diaminohexan.

Die Verbindung der Formel XVI

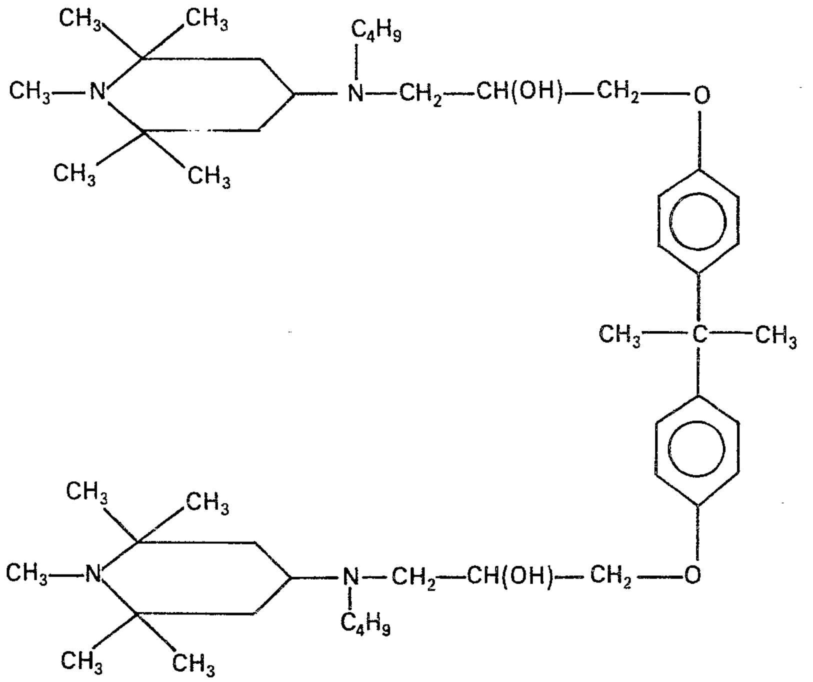

XVI

59) 4-(Bis-2-hydroxyäthyl)-amino-1,2,2,6,6-pentamethylpiperidin

60) 4-(3-Methyl-4-hydroxy-5-tert.butyl-benzoesäure-amido)-2,2,6,6-tetramethylpiperidin

61) 4-Methacrylamido-1,2,2,6,6-pentamethylpiperidin

62) α-Cyano-β-methyl-β-[N-(2,2,6,6-tetramethylpiperidin-4-yl)]-amino-acrylsäure-methylester

c) Lichtschutzmittel der Formel XVII

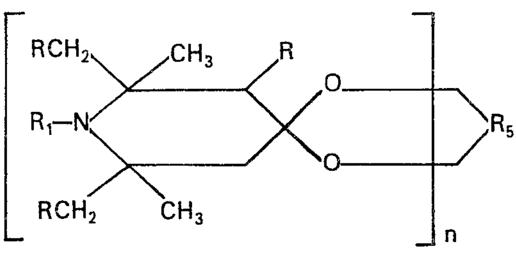

XVII

worin n die Zahlen 1 oder 2 bedeutet, R die für Formel XIII angegebene Bedeutung hat, $R_1$ die unter a) angegebene Bedeutung hat und $R_5$, wenn n = 1, $C_2$-$C_8$ Alkylen oder Hydroxyalkylen, $C_4$-$C_{22}$ Acyloxyalkylen, wenn n = 2, die Gruppe $(-CH_2)_2C(CH_2-)_2$ bedeutet.

Bedeutet $R_5$ $C_2$-$C_8$ Alkylen oder Hydroxyalkylen, so stellt es beispielsweise Äthylen, 1-Methyläthylen, Propylen, 2-Äthyl-propylen, 2-Äthyl-2-hydroxymethylpropylen dar.

Als $C_4$-$C_{22}$ Acyloxyalkylen bedeutet $R_5$ z.B. 2-Äthyl-2-acetoxy-methyl-propylen.

Beispiele für Polyalkylpiperidin-Lichtschutzmittel dieser Klasse sind folgende Verbindungen:

63) 9-Aza-8,8,10,10-tetramethyl-1,5-dioxaspiro-[5.5] undecan

64) 9-Aza-8,8,10,10-tetramethyl-3-äthyl-1,5-dioxaspiro[5.5] decan

65) 8-Aza-2,7,7,8,9,9-hexamethyl-1,4-dioxyspiro[4.5] decan

66) 9-Aza-3-hydroxymethyl-3-äthyl-8,8,9,10,10-pentamethyl-1,5-dioxaspiro[5.5] undecan

67) 9-Aza-3-äthyl-3-acetoxymethyl-9-acetyl-8,8,10,10-tetramethyl-1,5-dioxaspiro[5.5] undecan

68) 2,2,6,6-Tetramethylpiperidin-4-spiro-2'-(1',3'-dioxan)-5'-spiro-5''-(1'',3''-dioxan)-2''-spiro-4'''-(2''',2''',6''',6'''-tetramethylpiperidin).

d) Lichtschutzmittel der Formeln XVIIIA, XVIIIB und XVIIIC

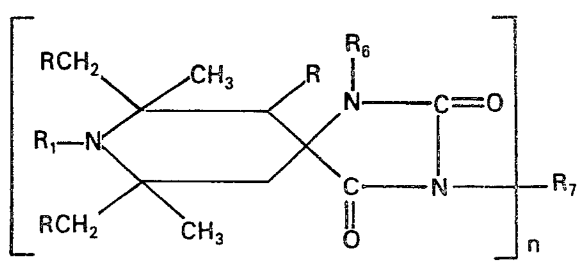

XVIIIA

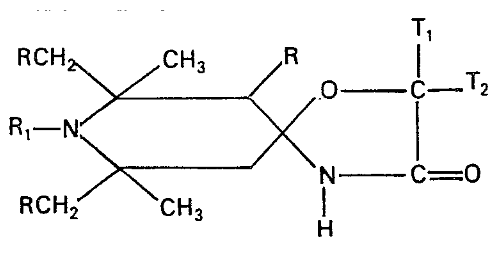

XVIIIB

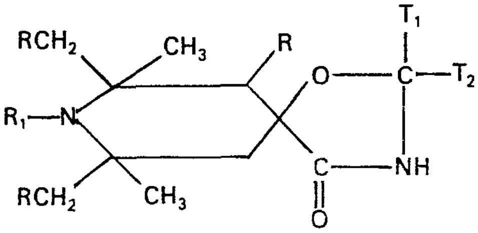

XVIIIC

worin n die Zahlen 1 oder 2 bedeutet, R die für Formel XIII angegebene Bedeutung hat, $R_1$ die unter a) angegebene Bedeutung hat, $R_6$ Wasserstoff, $C_1$-$C_{12}$ Alkyl, Allyl, Benzyl, Glycidyl, $C_2$-$C_6$ Alkoxyalkyl ist und $R_7$, wenn n = 1, Wasserstoff, $C_1$-$C_{12}$ Alkyl, $C_3$-$C_5$, Alkenyl, $C_7$-$C_9$ Aralkyl, $C_5$-$C_7$ Cycloalkyl, $C_2$-$C_4$ Hydroxyalkyl, $C_2$-$C_6$ Alkoxyalkyl, $C_6$-$C_{10}$ Aryl, Glycidyl, eine Gruppe der Formel $-(CH_2)_m$-COO-Q oder der Formel $-(CH_2)_m$-O-CO-Q, worin m 1 oder 2 und Q $C_1$-$C_{18}$ Alkyl oder Phenyl sind, wenn n = 2, $C_2$-$C_{12}$ Alkylen, $C_6$-$C_{12}$ Arylen, $C_4$-$C_{12}$ Alkenylen, eine Gruppe $-CH_2-(CH(OH)-CH_2-O-X-O-CH_2-CH(OH)-CH_2-$, worin X $C_2$-$C_{10}$ Alkylen, $C_6$-$C_{15}$ Arylen, $C_6$-$C_{12}$ Cycloalkylen ist, oder eine Gruppe $-CH_2CH(OZ')CH_2-(OCH_2-CH(OZ')CH_2)_2-$ bedeutet, worin Z' Wasserstoff, $C_1$-$C_{18}$ Alkyl, Allyl, Benzyl, $C_2$-$C_{12}$ Alkanoyl oder Benzoyl ist, $T_1$ und $T_2$ unabhängig voneinander Wasserstoff, $C_1$-$C_{18}$ Alkyl oder gegebenenfalls durch Halogen oder $C_1$-$C_4$ Alkyl substituiertes $C_6$-$C_{10}$ Aryl oder $C_7$-$C_9$ Aralkyl bedeuten oder $T_1$ und $T_2$ zusammen mit dem sie bindenden C-Atom gegebenenfalls durch $C_1$-$C_4$ Alkyl substituiertes $C_5$-$C_7$ Cycloalkyl, Pyrrolidinyl oder Piperperidinyl bilden.

Bedeuten etwaige Substituenten $C_1$-$C_{12}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl oder n-Dodecyl dar.

Etwaige Substituenten in der Bedeutung von $C_1$-$C_{18}$ Alkyl können z.B. die oben angeführten Gruppen und dazu noch beispielsweise n-Tridecyl, n-Tetradecyl, n-Hexadecyl oder n-Octadecyl darstellen.

Bedeuten etwaige Substituenten $C_2$-$C_6$ Alkoxyalkyl, so stellen sie z.B. Methoxymethyl, Äthoxymethyl, Propoxymethyl, tert.-Butoxymethyl, Äthoxyäthyl, Äthoxypropyl, n-Butoxyäthyl, tert.-Butoxyäthyl, Isopropoxyäthyl oder Propoxypropyl dar.

Stellt $R_7$ $C_3$-$C_5$ Alkenyl dar, so bedeutet es z.B. 1-Propenyl, Allyl, Methallyl, 2-Butenyl oder 2-Pentenyl.

Als $C_7$-$C_9$ Aralalkyl sind $R_7$, $T_1$ und $T_2$ insbesondere Phenäthyl oder vor allem Benzyl und als $C_5$-$C_7$ Cycloalkyl ($T_1$ und $T_2$ zusammen mit dem sie bindenden C-Atom) insbesondere Cyclohexyl.

Bedeutet $R_7$ $C_2$-$C_4$ Hydroxyalkyl, so stellt es z.B. 2-Hydroxyäthyl, 2-Hydroxypropyl, 2-Hydroxypropyl, 2-Hydroxybutyl oder 4-Hydroxybutyl dar.

Als $C_6$-$C_{10}$ Aryl bedeuten $R_7$, $T_1$ und $T_2$ insbesondere Phenyl, α- oder β-Naphthyl, die gegebenenfalls mit Halogen oder $C_1$-$C_4$ Alkyl substituiert sind.

Stellt $R_7$ $C_2$-$C_{12}$ Alkylen dar, so handelt es sich z.B. um Äthylen, Propylen, 2,2-Dimethylpropylen, Tetramethylen, Hexamethylen, Octamethylen, Decamethylen oder Dodecamethylen.

Als $C_4$-$C_{12}$ Alkenylen bedeutet $R_7$ insbesondere 2-Butenylen, 2-Pentenylen oder 3-Hexenylen.

Bedeutet $R_7$ $C_6$-$C_{12}$ Arylen, so stellt es beispielsweise o-, m- oder p-Phenylen, 1,4-Naphthylen oder 4,4'-Diphenylen dar.

Bedeutet Z' $C_2$-$C_{12}$ Alkanoyl, so stellt es beispielsweise Propionyl, Butyryl, Octanoyl, Dodecanoyl aber bevorzugt Acetyl dar.

X hat als $C_2$-$C_{10}$ Alkylen, $C_6$-$C_{15}$ Arylen oder $C_6$-$C_{12}$ Cycloalkylen die unter b) angegebene Bedeutung.

Beispiele für Polyalkylpiperidin-Lichtschutzmittel dieser Klasse sind folgende Verbindungen:

69) 3-Benzyl-1,3,8-Triaza-7,7,9,9-tetramethylspiro [4.5]decan-2,4-dion
70) 3-n-Octyl-1,3,8-triaza-7,7,9,9-tetramethylspiro[4.5]decan-2,4-dion
71) 3-Allyl-1,3,8-triaza-1,7,7,9,9-pentamethylspiro[4.5]decan-2,4-dion
72) 3-Glycidyl-1,3,8-triaza-7,7,8,9,9-pentamethylspiro[4.5]-decan-2,4-dion
73) 2-iso-Propyl-7,7,9,9-tetramethyl-1-oxa-3,8-diaza-4-oxo-spiro-[4,5]-decan
74) 2-Butyl-7,7,9,9-tetramethyl-1-oxa-3,8-diaza-4-oxo-spiro-[4,5]-decan
75) 2-Isopropyl-7,7,9,9-tetramethyl-1-oxa-4,8-diaza-3-oxo-spiro-[4,5]-decan
76) 2-Butyl-7,7,9,9-tetramethyl-1-oxa-4,8-diaza-3-oxo-spiro-[4,5]-decan,

sowie die folgenden Verbindungen:

77) 2,2,4,4-Tetramethyl-7-oxa-3,20-diaza-21-oxo-dispiro-[5,1,11,-2]-heneicosan
78) 1,3-Di(2,2,6,6-tetramethyl-4-piperidinyl)-imidazolidinon-2
79) 2,4,6-Tri[N-(2,2,6,6-tetramethyl-4-piperidinyl)-butylamino]-s-triazin
80) Di-[2-(2,2,6,6-tetramethyl-1-piperidino)-äthyl]-adipat
81) 2,2,6,6-Tetramethylpiperidin--1essigsäure-n-octylester.

Weitere Verbindungen der Formel:

82)

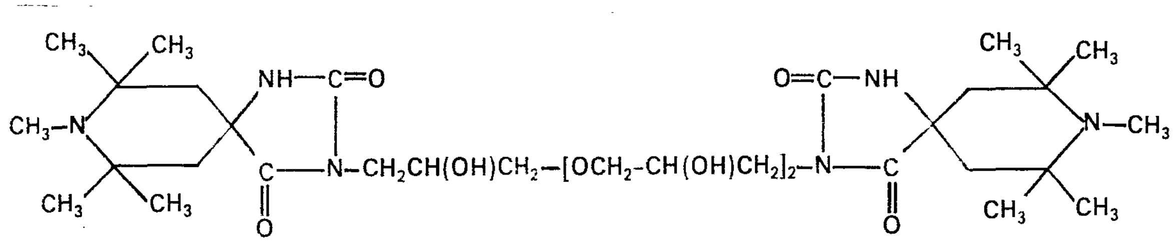

83)

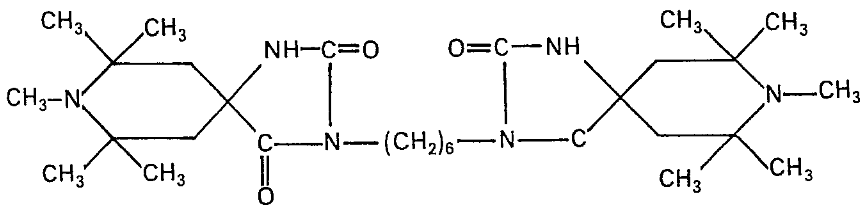

84)

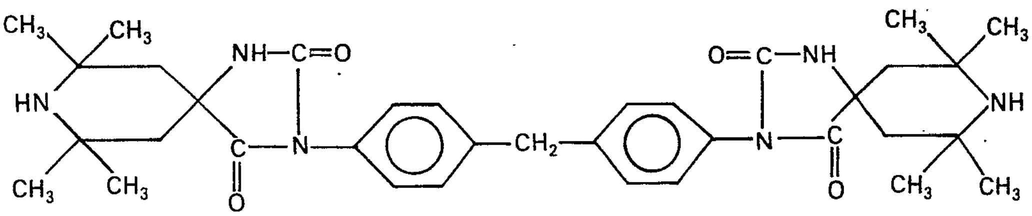

e) Lichtschutzmittel der Formel XIX

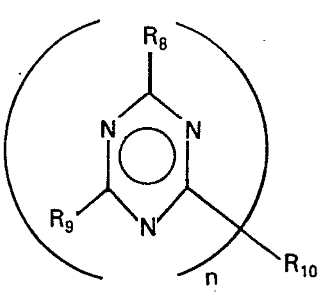

XIX

worin n die Zahlen 1 oder 2 ist und $R_8$ eine Gruppe der Formel

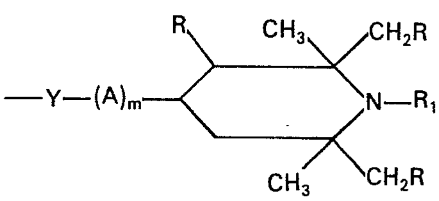

bedeutet, worin R die für Formel (I) angegebene Bedeutung hat, $R_1$ die unter a) angegebene Bedeutung hat, Y -O- oder $-NR_{11}-$ ist, A $C_2$-$C_6$ Alkylen oder $-(CH_2)_3-O-$ und m die Zahlen 0 oder 1 bedeuten, $R_9$ die Gruppen $R_8$, $NR_{11}R_{12}$, $-OR_{13}$,$-NHCH_2-OR_{13}$ oder $-N(CH_2OR_{13})_2$ ist, $R_{10}$ wenn n = 1 die Gruppen $R_8$ oder $R_9$ und wenn n = 1 die Gruppen $R_8$ oder $R_9$ und wenn n = 2 die Gruppe -Y-Q-Y-, worin Q gegebenenfalls durch $-N(R_{14})-$ unterbrochenes $C_2$-$C_6$ Alkylen bedeutet, $R_{11}$ $C_1$-$C_{12}$ Alkyl, Cyclohexyl, Benzyl oder $C_1$-$C_4$ Hydroxyalkyl oder eine Gruppe der Formel

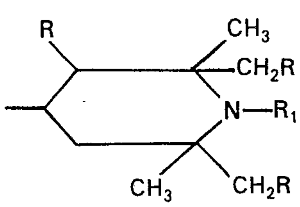

ist, $R_{12}$ $C_1$-$C_{12}$ Alkyl, Cyclohexyl, Benzyl, $C_1$-$C_4$ Hydroxyalkyl, $R_{13}$ Wasserstoff, $C_1$-$C_{12}$ Alkyl oder Phenyl und $R_{14}$ Wasserstoff, $C_1$-$C_{12}$ Alkyl, Cyclohexyl, Benzyl, Phenyl bedeuten oder $R_{11}$ und $R_{12}$ zusammen $C_4$-$C_5$ Alkylen oder Oxaalkylen sind oder auch $R_{11}$ und $R_{12}$ jeweils eine Gruppe der Formel

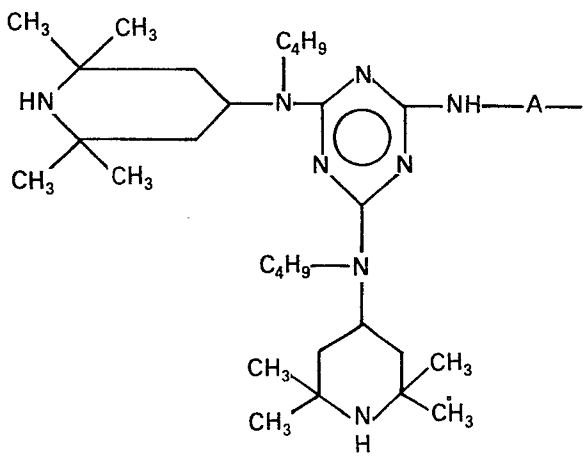

bedeuten.

Bedeuten etwaige Substituenten $C_1$-$C_{12}$ Alkyl, so stellen sie beispielsweise Methyl, Äthyl, n-Propyl, n-Butyl, sek.-Butyl, tert.-Butyl, n-Hexyl, n-Octyl, 2-Äthylhexyl, n-Nonyl, n-Decyl, n-Undecyl oder n-Dodecyl dar.

Bedeuten etwaige Substitutenten $C_1$-$C_4$ Hydroxyalkyl, so stellen sie z.B. 2-Hydroxyäthyl, 2-Hydroxypropyl, 3-Hydroxypropyl, 2-Hydroxybutyl oder 4-Hydroxybutyl dar.

Bedeutet A $C_2$-$C_6$ Alkylen, so stellt es beispielsweise Äthylen, Propylen, 2,2-Dimethylpropylen, Tetramethylen oder Hexamethylen dar.

Stellen $R_{11}$ und $R_{12}$ zusammen $C_4$-$C_5$ Alkylen oder Oxaalkylen dar, so bedeutet dies z.B. Tetramethylen, Pentamethylen oder 3-Oxa-pentamethylen.

Verbindungen der Formeln XX oder XXI

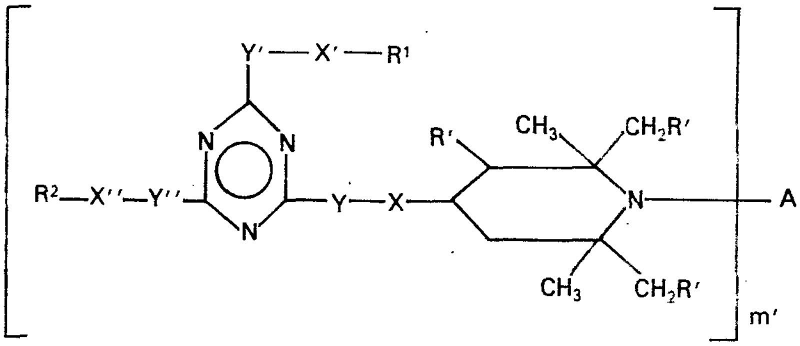

XX

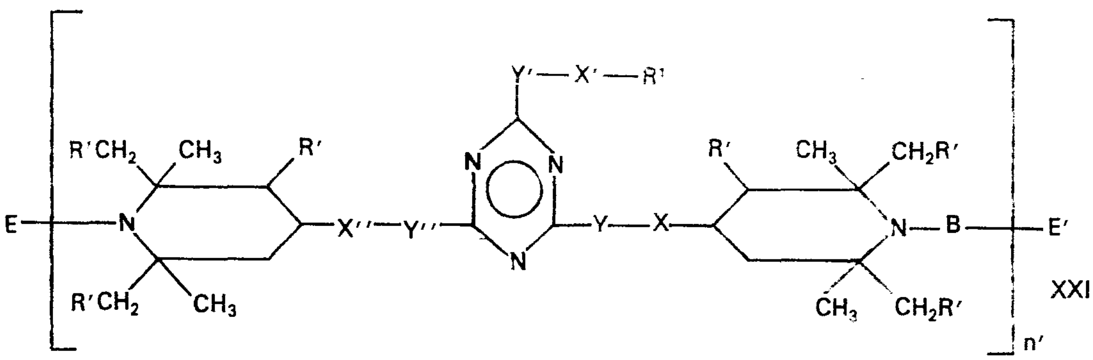

XXI

worin
m' 2, 3 oder 4 ist, n' 2 bis 50 ist,
X, X' und X'' eine direkte Bindung, $C_1$-$C_4$ Alkylen oder $-OCH_2CH_2CH_2-$, dessen O nicht an Y, Y' oder Y'' gebunden ist, darstellen
Y, Y' und Y'' -O-, -S-, -NH- oder $-NR^3-$ darstellen,
R' Wasserstoff oder $C_1$-$C_4$ Alkyl bedeutet,
$R^1$, $R^2$ und $R^3$ $C_1$-$C_{12}$ Alkyl, $C_2$-$C_8$ Alkoxyalkyl, $C_2C_4$ Hydroxyalkyl, $C_5$-$C_{10}$ Cycloalkyl, $C_6$-$C_{10}$ Aryl, durch 1 oder 2 $C_1$-$C_8$ Alkylgruppen und/oder OH und/oder $C_1$-$C_4$ Alkoxy substituiertes Phenyl oder eine Polyalkylpiperidinylgruppe der Formel darstellt

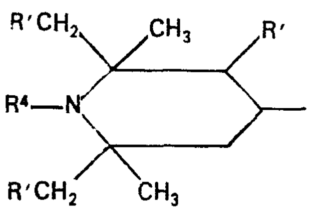

oder im Falle, dass Y' oder Y'' $-NR^3-$ ist und X' oder X'' eine direkte Bindung ist, $R^1$ und $R^3$ zusammen mit dem N-Atom einen Pyrrolidin-, Piperidin- oder Morpholinring bilden,
$R^4$ Wasserstoff, O·, $C_1$-$C_{12}$ Alkyl, Allyl oder Benzyl bedeutet,
A, wenn m 2 ist, $C_2$-$C_{12}$ Alkylen, $C_4$-$C_8$ Alkenylen, Xylylen oder einen Rest der Formel
$-CH_2-COO-R^5-OOC-CH_2-$, $-CH_2-CH(OH)-CH_2-$ oder
$-CH_2CH(OH)CH_2-D-CH_2CH(OH)CH_2-$ darstellt,
wenn m 3 ist,
eine Gruppe der Formel

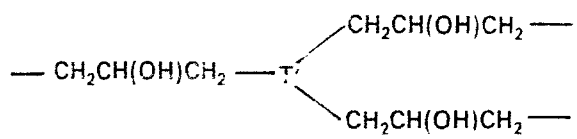

darstellt und
wenn m 4 ist, eine Gruppe der Formel

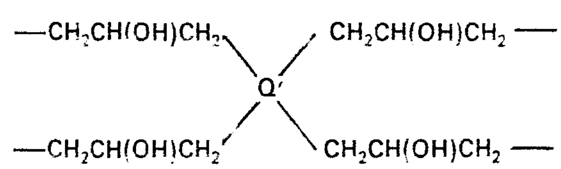

darstellt,
B $C_2$-$C_{12}$ Alkylen, $C_4$-$C_8$ Alkenylen, Xylylen oder einen Rest der Formel $-CH_2-COO-R^5-OOC-CH_2-$, $-CH_2CH(OH)-CH_2-$ oder $-CH_2CH(OH)CH_2-D-CH_2CH(OH)CH_2-$ darstellt,
$R^5$ $C_2$-$C_8$ Alkylen, $C_4$-$C_8$ Oxaalkylen oder Cyclohexylen darstellt,
D einen zweiwertigen Rest der Formel $-O-R^6-O-$, $-O-C(O)-R^7-C(O)-O-$, $-OCH(R^8)CH_2O-R^6-OCH_2CH(R^8)O-$ oder

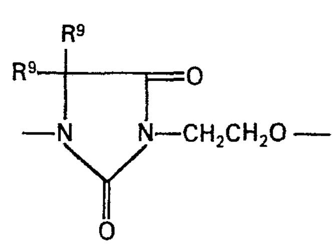

darstellt,
$R^6$ $C_2$-$C_{12}$ Alkylen, $C_6$-$C_{12}$ Cycloalkylen, $C_6$-$C_{12}$ Arylen oder -Phenylen-Z'-Phenylen- darstellt, worin Z' $-CH_2-$, $>C(CH_3)_2$, $-SO_2-$ oder -O- bedeutet,
$R^7$ eine direkte Bindung, $C_1$-$C_{12}$ Alkylen, $C_2$-$C_6$ Alkenylen, $C_6$-$C_{12}$ Arylen darstellt,
$R^8$ und $R^9$ Wasserstoff oder $C_1$-$C_4$ Alkyl sind,
T' einen dreiwertigen Rest der Formeln

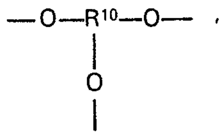 , 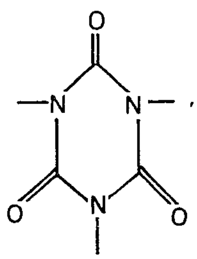 ,

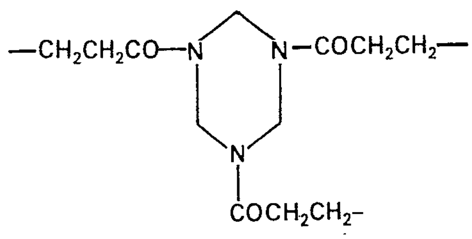

oder

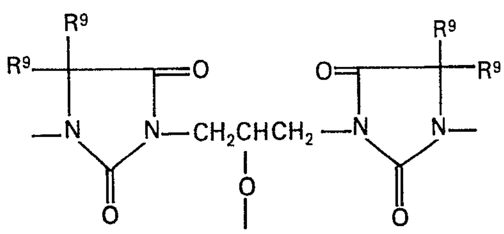

darstellt,
$R^{10}$ ein dreiwertiger aliphatischer Kohlenwasserstoffrest mit 3–10 C-Atomen ist,
Q' einen vierwertigen Rest der Formel

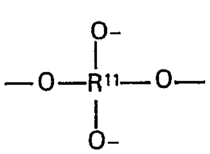

oder

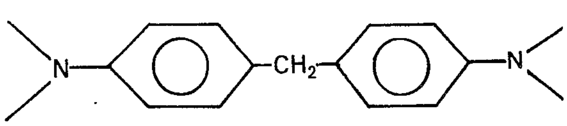

darstellt,
$R^{11}$ einen vierwertigen aliphatischen Kohlenwasserstoffrest mit 4–10 C-Atomen bedeutet und

E und E' Endgruppen darstellen.

$R^1$, $R^2$, $R^3$ und $R^4$ als $C_1$-$C_{12}$ Alkyl können dabei verzweigte oder unverzweigte Alkylreste sein, z.B. Methyl, Äthyl, Isopropyl, tert. Butyl, Hexyl, Isooctyl, Decyl oder Dodecyl.

$R^1$, $R^2$, $R^3$ als Alkoxyalkyl können z.B. Methoxymethyl, 2-Methoxyäthyl, 2-Äthoxyäthyl, 2-Isopropoxyäthyl, 2-n-, sec.- oder tert. Butoxyäthyl oder 2-Butoxypropyl sein.

$R^1$, $R^2$, $R^3$ als Hydroxyalkyl können z.B. 2-Hydroxyäthyl, 2-Hydroxyäthyl, 2-Hydroxypropyl, 2-Hydroxybutyl oder 3-Hydroxypropyl sein.

$R^1$, $R^2$, $R^3$ als $C_5$-$C_{10}$ Cycloalkyl können beispielsweise Cyclopentyl, Cylcohexyl, 3-Methylcyclohexyl oder 4-tert. Butylcyclohexyl sein.

$R^1$, $R^2$, $R^3$ als $C_6$-$C_{10}$ Aryl können Phenyl oder Naphthyl sein, wobei Phenyl bevorzugt ist.

$R^1$, $R^2$, $R^3$ als substituiertes Phenyl, können z.B. p.Tolyl, 4-Hydroxyphenyl, 4-tert.-Butylphenyl oder 3,5-Di-tert.-butyl-4-hydroxyphenyl sein.

X als $C_1$-$C_4$ Alkylen kann beispielsweise Methylen, Äthylen, 1,3-Propylen, 1,2-Propylen, 1,1-Dimethyläthylen oder 2,2-Propylen sein.

A, B oder $R^6$ als Alkylen kann eine verzweigte oder unverzweigte Alkylengruppe sein, wie z.B. Äthylen, Tri-, Tetra-, Hexa-, Octa-, Deca- oder Dodecamethylen, 2,2-Dimethylpropylen-1,3, 1,2-Butylen oder 1,2-Propylen. $R^5$ ist ein zweiwertiger aliphatischer oder cycloaliphatischer Rest, beispielsweise Äthylen, 1,2-Propylen, 1,2-Butylen, 1,3-Propylen, 1,4-Butylen, 1,6-Hexylen, 1,4-Cyclohexylen oder 3-Oxapentylen-1,5.

A und B als Alkenylen können z.B. 1,4-Buten-2-ylen oder 1,6-Hexen-3-ylen sein.

$R^6$ als Cycloalkylen kann z.B. 1,4-Cyclohexylen oder 1,4-Cyclooctylen sein. $R^6$ als Arylen kann Phenylen, Naphthylen oder Diphenylen sein.

$R^7$ als Alkylen oder Alkenylen kann z.B. Methylen, 1,3-Propylen, Tetramethylen, 2,2-Dimethyl-1,3-propylen, Octamethylen, Dodecamethylen, Vinylen oder 1,4-Buten-2-yeln sein. $R^7$ als cyclischer Rest kann beispielsweise 1,2-Cyclopentylen, 1,2-Cyclohexylen, 1,2-Cyclohexen-4-ylen, 3,6-Endomethylencyclohexen-4-ylen-1,2, 1,2-Phenylen, 1,4-Phenylen oder 1,4-Naphthylen sein.

Beispiele für Polyalkylpiperidin-Lichtschutzmittel dieser Klasse sind die Verbindungen der folgenden Formeln:

85) 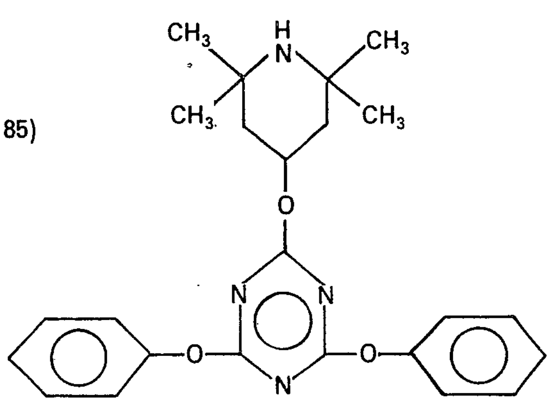

86)

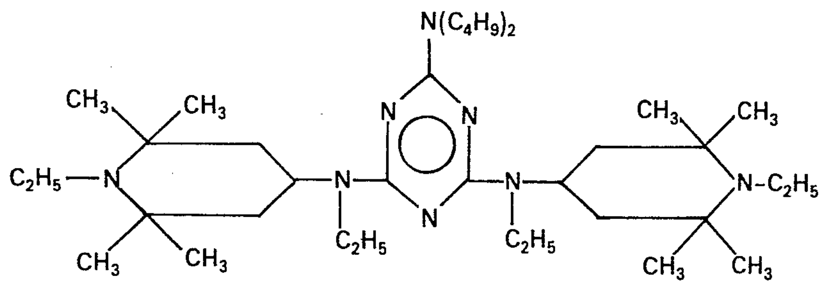

87)

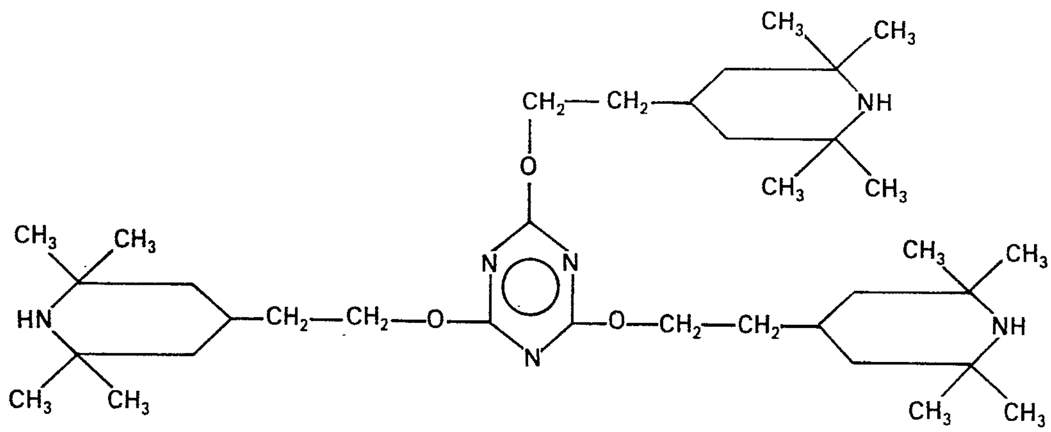

88)

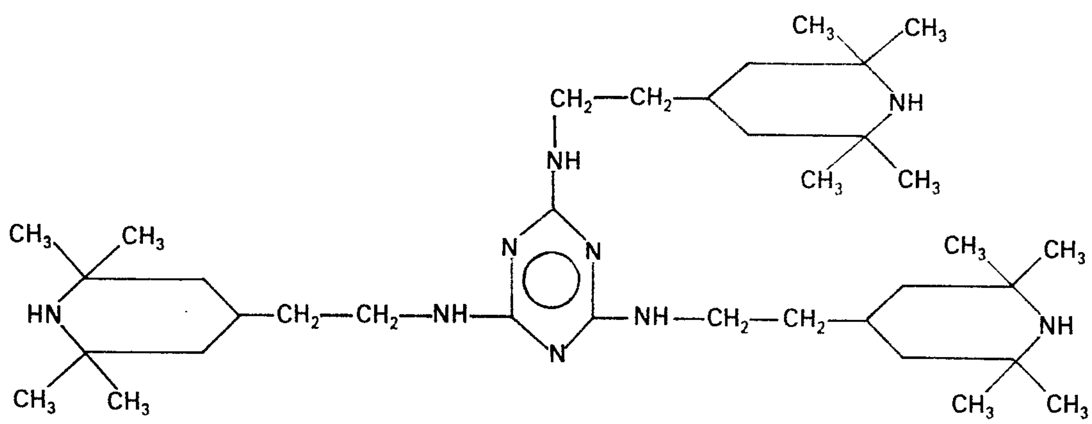

88a)

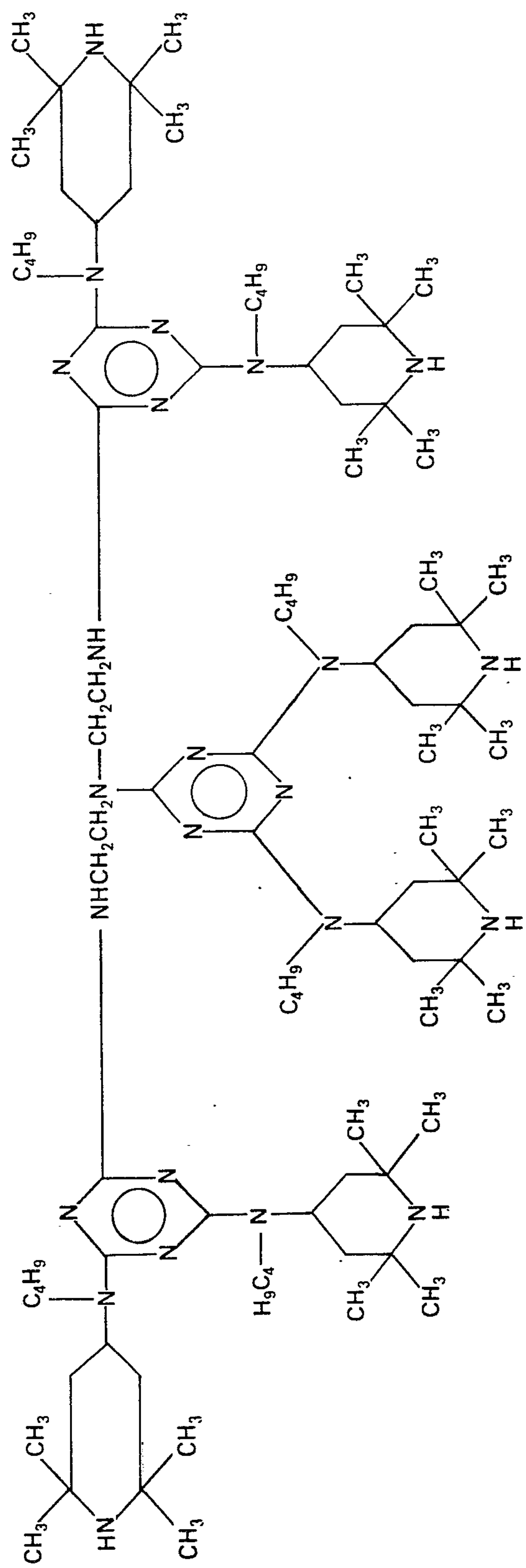

89)

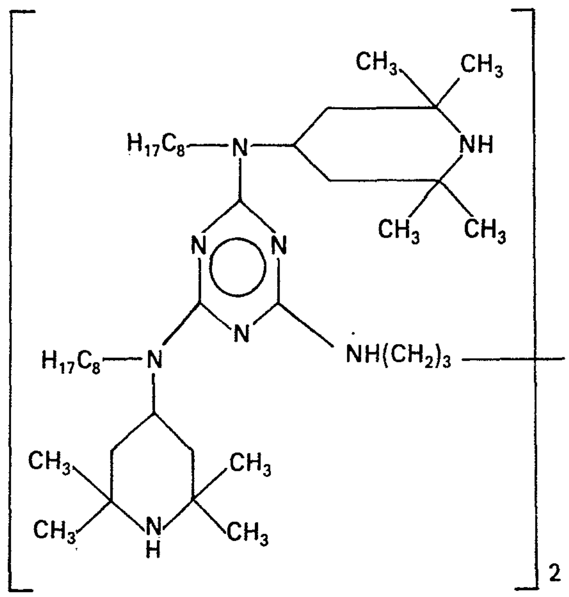

90)

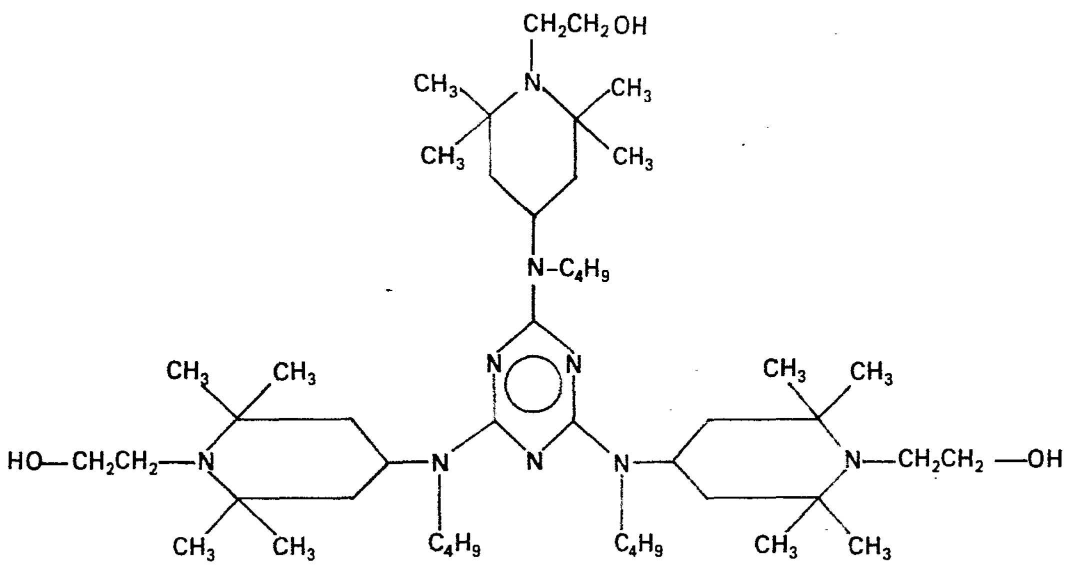

g) Lichtschutzmittel der Formel XXII

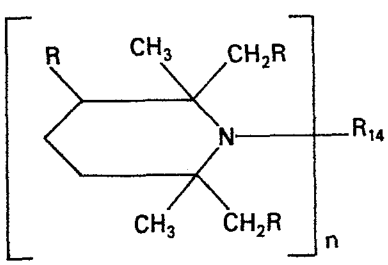

XXII

worin n die Zahlen 1 oder 2 bedeutet, R die für Formel XIII angegebene Bedeutung hat und $R_{14}$, wenn n = 1, $C_4$-$C_{18}$ Alkyl, $C_7$-$C_{12}$ Aralkyl, die Gruppe -CO-$R_{15}$, $C_1$-$C_4$ Alkyl substituiert durch -CH, -COO$R_{16}$k -OH, -OCO$R_{17}$ oder

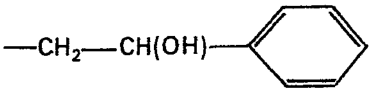

bedeutet, wobei $R_{15}$ $C_1$-$C_{12}$ Alkyl, $C_2$-$C_4$ Alkenyl oder Phenyl, $R_{16}$ $C_1$-$C_{18}$ Alkyl, $R_{17}$ $C_1$-$C_{18}$ Alkyl, $C_2$-$C_{10}$ Alkenyl, Cyclohexyl, Benzyl oder $C_6$-$C_{10}$ Aryl sind oder, wenn n = 2, $R_{14}$ $C_4$-$C_{12}$ Alkylen, 2-Butenylen-1,4, Xylylen, die Gruppe -$CH_2)_2$-OOC-$R_{18}$-COO-$(CH_2)_2$- oder die Gruppe -$CH_2$-OOC-$R_{19}$-COO-$CH_2$- ist, wobei $R_{18}$ $C_2$-$C_{10}$ Alkylen, Phenylen oder Cyclohexylen und $R_{19}$ $C_2$-$C_{10}$ Alkylen, Xylylen oder Cyclohexylen bedeuten.

Bedeuten etwaige Substituenten $C_1$-$C_{12}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl oder n-Dodecyl dar.

Etwaige Substituenten, die $C_1$-$C_{18}$ Alkyl bedeuten, können z.B. die die oben angeführten Gruppen und dazu noch beispielsweise n-Tridecyl, n-Tetradecyl, n-Hexadecyl oder n-Octadecyl darstellen.

Stellen etwaige Gruppen $C_2$-$C_{10}$ Alkylen dar, so

bedeuten sie beispielsweise Äthylen, Propylen, 2,2-Dimethylpropylen, Tetramethylen, Hexamethylen, Octamethylen oder Decamethylen.

$R_{14}$ bedeutet als $C_4$-$C_{18}$ Alkyl, z.B. n-Butyl, sek.-Butyl, tert.-Butyl, n-Hexyl, n-Octyl, 2-Äthyl-hexyl, 1,1-Dimethyl-2-tert.-butyläther, n-Nonyl, n-Decyl, n-Dodecyl, n-Tridecyl, n-Tetradecyl, n-Hexadecyl oder n-Octadecyl.

Bedeutet $R_{14}$ ein durch -CN substituiertes $C_1$-$C_4$ Alkyl, so stellt es beispielsweise Cyanomethyl, Cyanoäthyl, 3-Cyano-n-propyl, 4-Cyano-n-butyl dar.

Bedeutet $R_{14}$ $C_4$-$C_{12}$ Alkylen, so handelt es sich z.B. um 2,2-Dimethylpropylen, Tetramethylen, Hexamethylen, Octamethylen, Decamethylen oder Dodecamethylen.

Stellt $R_{14}$ $C_7$-$C_{12}$ Aralkyl dar, so bedeutet es insbesondere Phenäthyl, p-Methyl-benzyl oder vor allem Benzyl.

$R_{15}$ bedeutet als $C_2$-$C_4$ Alkenyl beispielsweise Vinyl, 1-Propenyl, Allyl, Methallyl, 2-Butenyl.

$R_{17}$ bedeutet als $C_2$-$C_{10}$ Alkenyl z.B. die für $R_{15}$ in der Bedeutung Alkenyl angeführten Gruppen und dazu noch beispielsweise Crotyl, 2-Hexenyl, 2-Octenyl oder 2-Decenyl.

Stellt $R_{17}$ $C_6$-$C_{10}$ Aryl dar, so bedeutet es beispielsweise gegebenenfalls in o- oder p-Stellung mit Methyl, Äthyl, Isopropyl, n-Butyl oder tert.-Butyl substituiertes Phenyl.

Beispiele für Polyalkylpiperidin-Lichtschutzmittel dieser Klasse sind folgende Verbindungen:

91) Bis-[β-(2,2,6,6-tetramethylpiperidion)-äthyl]-sebacat

92) α-(2,2,6,6-Tetramethyl-piperidion)-essigsäure-n-octylester

93) 1,4-Bis-(2,2,6,6-tetramethylpiperidino)-2-buten.

h) Lichtschutzmittel der Formel XXIII

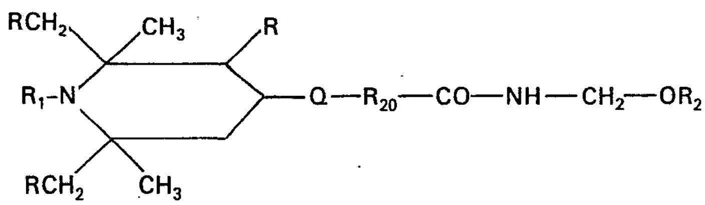

XXIII

worin Q -N($R_3$)- oder -O- ist, $R_{20}$ $C_1$-$C_3$ Alkylen, die Gruppe -$CH_2$-CH ($R_4$)-O-, worin $R_4$ Wasserstoff, Methyl oder Phenyl ist, die Gruppe -$(CH_2)_3$-NH- oder eine Einfachbindung bedeutet, R Wasserstoff oder Methyl ist, $R_1$ die unter a) angegebene Bedeutung hat, $R_2$ Wasserstoff oder $C_1$-$C_{18}$ Alkyl bedeutet, $R_3$ Wasserstoff, $C_1$-$C_{18}$ Alkyl, $C_5$-$C_7$ Cycloalkyl, $C_7$-$C_{12}$ Aralkyl, Cyanäthyl, $C_6$-$C_{10}$ Aryl, die Gruppe -$CH_2$-CH($R_4$)-OH, worin $R_4$ die oben angegebene Bedeutung hat, eine Gruppe der Formel:

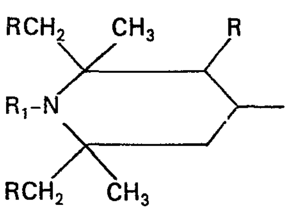

oder eine Gruppe der Formel

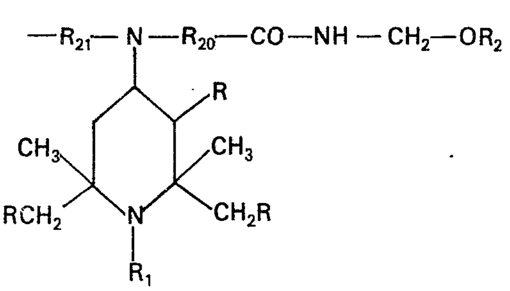

bedeutet, worin $R_{21}$ $C_2$-$C_6$ Alkylen oder $C_6$-$C_{12}$ Arylen sein kann, oder $R_3$ eine Gruppe -$R_{20}$-CO-NH-$CH_2$-$OR_2$ ist.

Bedeuten etwaige Substituenten $C_1$-$C_{18}$ Alkyl, so stellen sie z.B. Methyl, Äthyl, n-Propyl, n-Butyl, sek.-Butyl, tert.-Butyl, n-Hexyl, n-Octyl, 2-Äthyl-hexyl, n-Nonyl, n-Decyl, n-Undecyl, n-Dodecyl, n-Tridecyl, n-Tetradecyl, n-Hexadecyl oder n-Octadecyl dar.

Stellen etwaige Substituenten $C_7$-$C_{12}$ Aralkyl dar, so bedeuten sie beispielsweise Phenäthyl oder insbesondere Benzyl.

Wenn $R_1$ $C_3$-$C_8$ Alkenyl bedeutet, so kann es sich z.B. um 1-Propenyl, Allyl, Methallyl, 2-Butenyl, 2-Pentenyl, 2-Hexenyl, 2-Octenyl oder 4-tert.-Butyl-2-butenyl handeln.

$R_1$ ist als $C_3C_8$ Alkinyl bevorzugt Propargyl. Als $C_1$-$C_8$ Alkanol bedeutet $R_1$ beispielsweise Formyl, Propionyl, Butyryl, Octanoyl aber bevorzugt Acetyl und als $C_3$-$C_5$ Alkenoyl, insbesondere Acryloyl.

$R_3$ bedeutet als $C_5$-$C_7$ Cycloalkyl, insbesondere Cyclohexyl.

Als $C_6$-$C_{10}$ Aryl bedeutet $R_3$ insbesondere Phenyl α- oder β-Naphthyl, die gegebenenfalls mit Halogen oder $C_1$-$C_4$ Alkyl substituiert sind. E bedeutet als $C_1$-$C_3$ Alkylen z.B. Methylen, Äthylen oder Propylen.

$R_{21}$ stellt als $C_2$-$C_6$ Alkylen beispielsweise Äthylen, Propylen, 2,2-Dimethylpropylen, Tetramethylen oder Hexamethylen und als $C_6$-$C_{12}$ Arylen o-, m- oder p-Phenylen, 1,4-Naphthylen oder 4,4'-Diphenylen dar.

Beispiele für Polyalkylpiperidin-Lichtschutzmittel dieser Klasse sind folgende Verbindungen:

94) N-Hydroxymethyl-N'-2,2,6,6-tetramethyl-piperidin-4-yl-harnstoff

95) N-Methoxymethyl-N'-2,2,6,6-tetramethyl-piperidin-4-yl-harnstoff

96) N-Methoxymethyl-N'-n-docecyl-N'-2,2,6,6-tetramethyl-piperidin-4-yl-harnstoff
97) O-(2,2,6,6-Tetramethylpiperidin-4-yl)-N-methoxy-methylurethan.

i) Polymere Verbindungen, deren wiederkehrende Struktureinheit einen Polyalkylpiperidinrest der Formel (I) enthält, insbesondere Polyester, Polyäther, Polyamide, Polyamine, Polyurethane, Polyharnstoffe, Polyaminotriazine, Poly(meth)acrylate, Poly(meth)acrylamide und deren Copolymeren, die solche Reste enthalten.

98) Polyester aus Bernsteinsäure, Sebacinsäure, Di-butylmalonsäure, Oxalsäure oder Isophthalsäure und 1-Hydroxyäthyl-2,2,6,6-tetramethyl-4-hydroxypiperidin
99) Polyamid aus Bernsteinsäure, Adipinsäure oder Phthalsäure und 1-(3-Aminopropyl)-2,2,6,6-tetramethyl-4-aminopiperidin
100) Polyamid aus Oxalsäure oder p-Phenylendiessigsäure und N,N'-bis(1,2,2,6,6-pentamethyl-4-piperidinyl)-1,6-diaminohexan
101) Polyamin aus 1,2,2,6,6-Pentamethyl-4-aminopiperidin und Epichlorhydrin oder Bisphenol-A-diglycidäther
102) Polytriazin aus 2,4-Dichlor-6-N-(1,2,2,6,6-pentamethyl-4-piperidinyl)äthylamino-s-triazin und N,N'-Bis(2,2,6,6-tetramethyl-4-piperidinyl)-1,6-diaminohexan
103) Copolymerisat aus 1-Benzyl-2,2,6,6-tetramethyl-4-acryl-amidopiperidin und N-Butylacrylamid.

Beispiel für Polyalkylpiperidin-Lichtschutzmittel dieser Klasse sind die Verbindungen der folgenden Formeln, wobei m die Zahlen 2- etwa 200 bedeutet.

104) 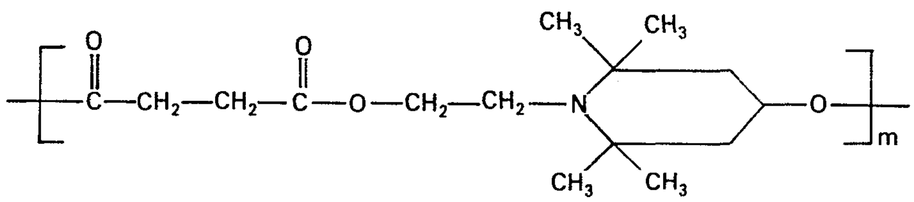

105)

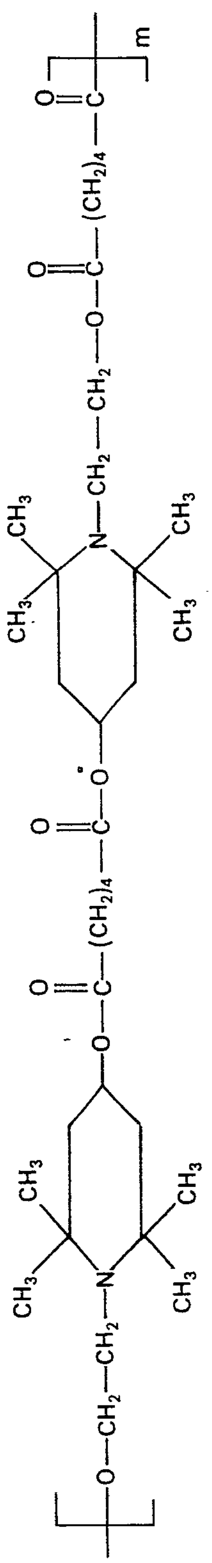

106)

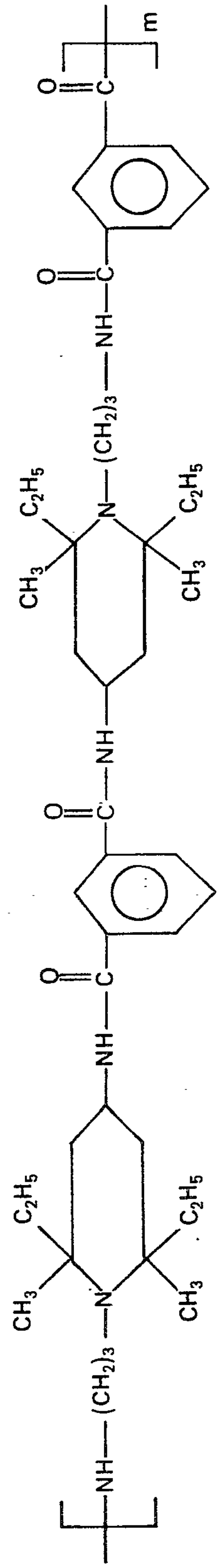

107)

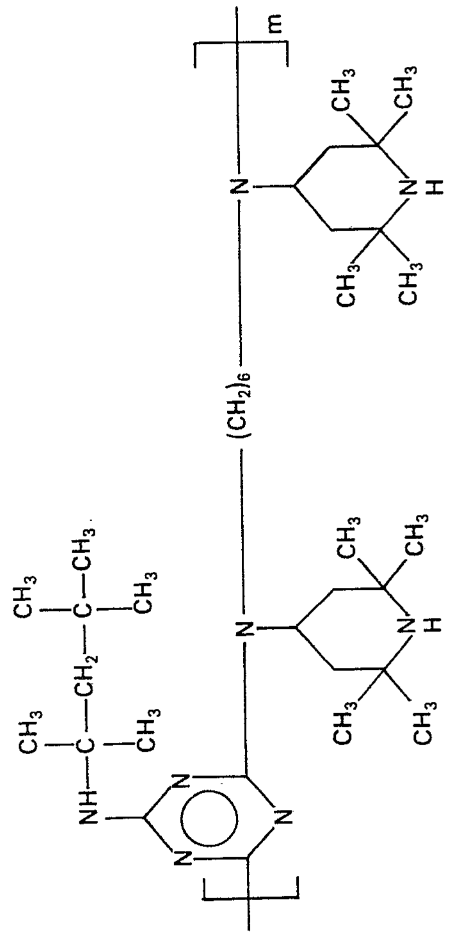

108) 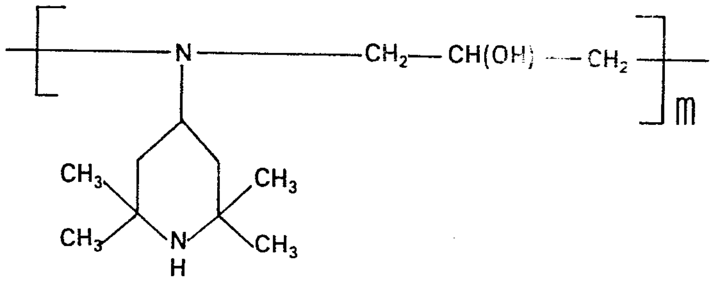

109) 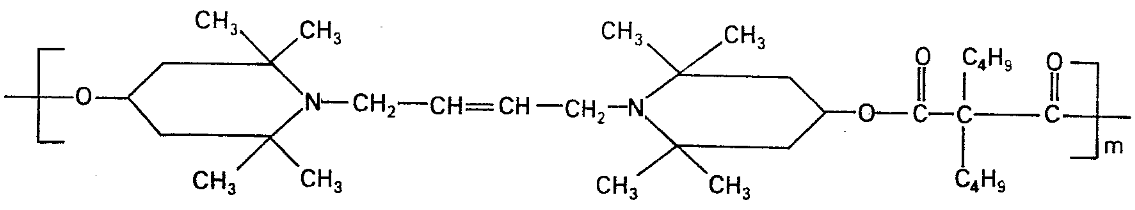

110) 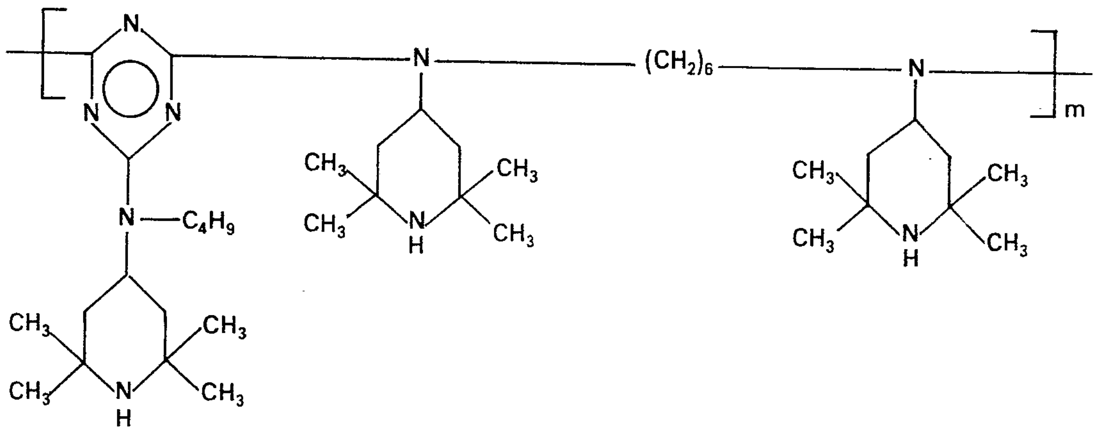

111) 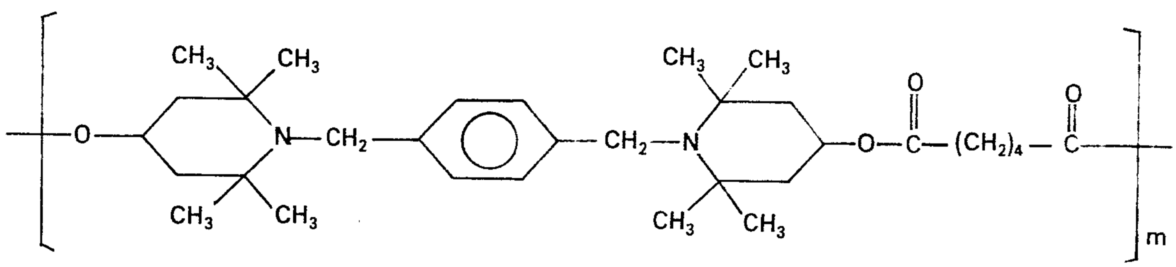

112) 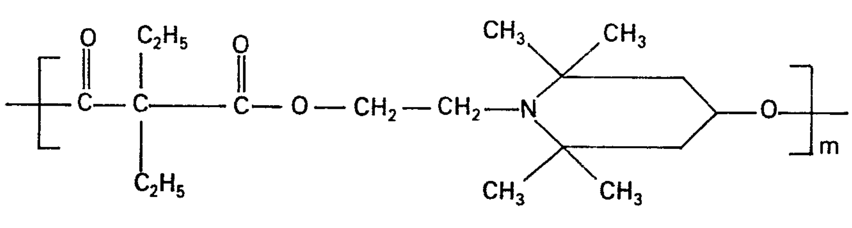

113)

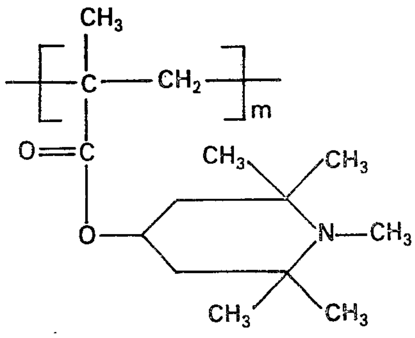

114)

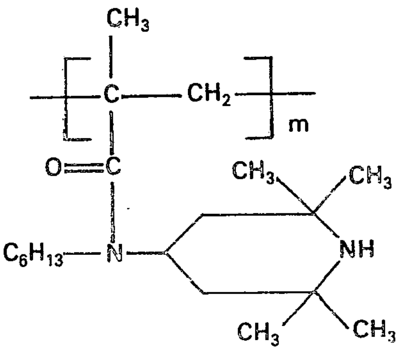

j) Verbindungen, die in ihrem Molekül mindestens eine 2-(2'-Hydroxyphenyl)-benztriazol-Gruppe oder 2-Hydroxybenzophenon-Gruppe und mindestens eine Polyalkylpiperidingruppe enthalten.

Beispiele für Polyalkylpiperidinlichtschutzmittel dieser Klasse sind die Verbindungen der folgenden Formeln

115)

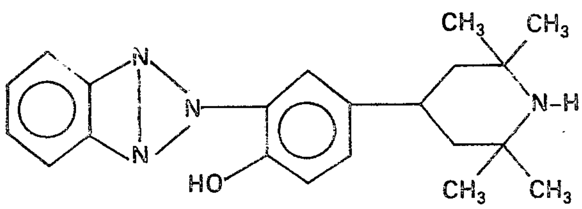

116)

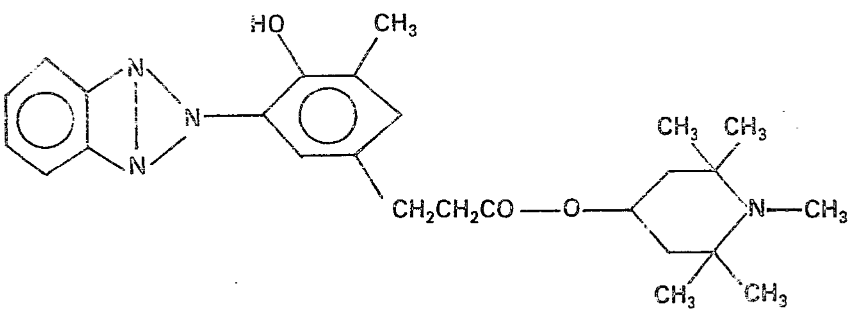

117)

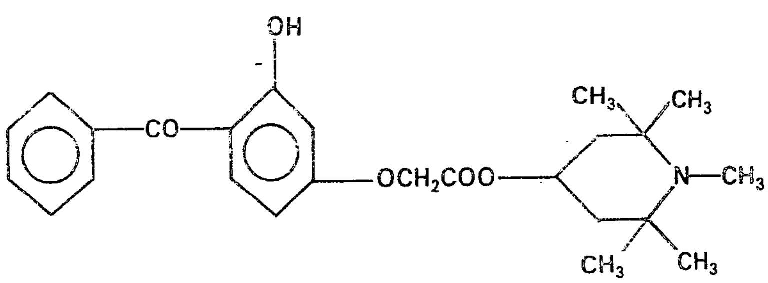

118)

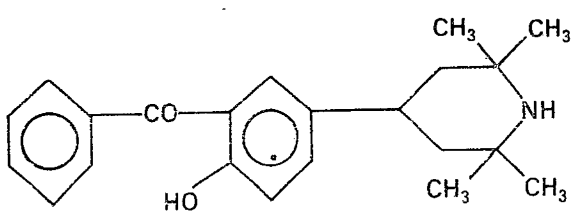

Es können auch Polyalkylpiperidinderivate der Klasse a)-j) verwendet werden, die mit dem Bindemittel des Lackes chemische Bindungen eingehen. Dies ist der Fall, wenn das Polyalkylpiperidinderivat eine hierfür geeignete reaktive Gruppe besitzt, wie beispielsweise eine Glycidyl- oder eine Methylolgruppe.

Beispiele solcher Verbindungen sind die Methylol- bzw. Methyloläthergruppen enthaltende Polyalkylpiperidinderivate der Klasse h).

Soweit die Polyalkylpiperidinverbindungen basische Verbindungen sind, können sie mit Säuren Salze bilden. Hierfür kommen beispielsweise anorganische Säuren oder organische Carbon-, Sulfon-, Phosphon- oder Phospinsäuren in Frage, wie z.B. Chlorwasserstoffsäure, Borsäure, Phosphorsäure, Essigsäure, Salicylsäure, Toluolsulfonsäure oder Benzolphosphonsäure.

Die Polyalkylpiperidinverbindungen können mit komplexbildenden Metallverbindungen Komplexe bilden, beispielsweise mit Zink-II-acetat, Cobalt-II-acetylacetonat, Nickel-II-acetylacetonat, Aluminium-III-acetylacetonat, Nickel-II-benzoat oder Aluminium-III-benzoylacetonat.

Salz aus 1 Mol $H_3PO_4$ und 1 Mol Adipinsäure-di-(1,2,2,6,6-pentamethyl-4-piperidinyl)-ester

Salz aus 2 Mol Bis(3,5-di-tert.-butyl-4-hydroxybenzyl)-malonsäure und 1 Mol 2,2,6,6-Tetramethyl-4-lauroyl-oxypiperidin 1:1-Komplex von Bis-(2,2,6,6-tetramethyl-4-piperidinyl)-sebacat und Nickel-II-acetylacetonat

1:2-Komplex von Bis-(1,2,2,6,6-pentamethyl-4-piperidinyl)-adipat und Nickel-II-acetat.

Die folgenden Beispiele erläutern die Erfindung. Prozente bedeuten Gewichtsprozente.

A. Herstellung des Vorproduktes: 2,4-Di-(α,α-dimethyl-benzyl)phenol

Dieses Zwischenprodukt wurde nach der Beschreibung in US-PS 2 714 120 hergestellt. 705,8 g (7,5 Mol) Phenol wird mit 1772,7 g (15 Mol) α-Methylstyrol in Gegenwart von 25,7 g (0,135 Mol) p-Toluolsulfonsäure Monohydrat als Katalysator vermischt und während 2,5 Std. auf 140°C unter $N_2$ erhitzt. Nach Beendigung der Reaktion kühlt man das Gemisch auf 110°C ab und fügt 1125 ml Toluol zu. Dann wäscht man die resultierende Lösung bei 80°C mit 750 ml wässriger Lösung bestehend aus 37,5 g $NaCO_3$ und 75 g NaCl. Die organische Phase wäscht man dreimal mit 1000 ml wässriger NaCl-Lösung, trocknet über wasserfreiem Natriumsulfat und anschliessend isoliert man durch Filtrieren und Vakuumdestillieren.

Die Hauptfraktion enthält 1229,8 g des Produktes (49,6%
der Theorie). Sdp. 172–175°/0,15–018 mm/Hg. Smp. 63–65°C.

B. Vergleichsbeispiel: Herstellung des Zwischenproduktes 2-Nitro-2'-hydroxy-3',5'-di(α,α-dimethyl-benzyl)azobenzol durch saure Kupplung

In einen 2 l Dreihals-Rundkolben mit Rührer und Thermometer gibt man 90,6 g einer 26%igen wässrigen Naphtalinsulfonsäure-Lösung, 1,9 g Triton X-207 (nicht ionischer Oberflächenaktiver Stoff), 5,6 g Conoco AAS-90F (Natrium-dodecylbenzol-sulfonat) und 90 ml Wasser. Das Gemisch wird auf 40°C erwärmt, dann mit 16,5 g 2,4-Di-(α,α-di-methyl-benzyl)phenol, welches auf 90°C vorgeheizt wurde, versetzt und unter kräftigem Rühren auf 40°C gehalten.

Eine kalte o-Nitrobenzoldiazoniumchlorid-Lösung wird aus 49,8 g (0,36 Mol) o-Nitroanilin und aus 24,9 g (0,36 Mol) Natriumnitrit in conc. HCl bei −5°C bis 0°C hergestellt. Diese Lösung wird tropfenweise während 3 Stunden zum Reaktionsgemisch gegeben und die resultierende dunkelrote bis schwarze Lösung über Nacht bei 40°C gehalten. Dann wird die Temperatur für eine Stunde auf 65°C und auf weitere 30 Minuten auf 95°C erhöht. Nach Abkühlen auf 85°C, wird das Rohprodukt, eine tiefrote viskose Masse, isoliert.

Das zerkleinerte Rohprodukt wird mit 200 ml heissem Wasser (75°C) vermengt und anschliessend mit 400 ml Methanol über Nacht stehengelassen. Das Gemisch wird mit weiteren 400 ml Methanol zu einem feinen Granulat gerührt. Es ergab 81,9 g (48,4% der Theorie) des Zwischenproduktes. Smp: 139–141°C.

Das Dünnschichtchromatogramm zeigt ein homogenes Produkt mit $R_f$ = 0,61 auf Silicagel in 3 Teilen Cyclohexan und 1 Teil Äthylacetat als Lösungsmittel

Beispiel 1:

2-[2-Hydroxy-3,5-di(α,α-dimethyl-benzyl)-phenyl]-2H-benzotriazol.

In einen 5 l Dreihals-Rundkolben mit Gaseinleitung, Rührer und Rückflusskühler, gibt man 386 g (0,805 Mol) o-Nitroazobenzol, hergestellt im Beispiel A, und 1200 ml Toluol. Zu dieser Lösung fügt man 240 ml Isopropanol und 240 ml Wasser. Unter Einleiten von Stickstoff fügt man 160 ml 50,1% Natronlauge zur Lösung. Eine Flasche mit 158,2 g (2,42 Grammatom) Zink wird an den Reaktionskolben angeschlossen. Man leitet den Zinkstaub portionenweise während 90 Minuten in das Reaktionsgemisch, und zwar so, dass die Reaktionstemperatur zwischen 40–45°C bleibt. Nach Beendigung der Zinkzugabe hält man das Reaktionsgemisch auf 40°C 1 Stunde lang und erwärmt dann auf 70°C 3 Stunden lang. Das Gemisch wird auf Raumtemperatur abgekühlt und mit 600 ml konz. Salzsäure angesäuert.

Der Zink-Rückstand wird durch Filtrieren entfernt. Die organische Phase, welche das Produkt enthält, wird viermal mit 340 ml verdünnter Salzsäure gewaschen. Nach Entfernen des Lösungsmittels durch Vakuumdestillation erhält man das viskose Rohprodukt, das beim Stehen kristallisiert.

Um das Rohprodukt zu reinigen, rekristallisiert man es zuerst aus 750 ml Äthylacetat und dann aus 1000 ml Acetonitril/Äthylacetat Gemisch (4:1) und löst anschliessend in 1250 ml Toluol. Die Toluol-Lösung wird mit 70% wässriger Schwefelsäure extrahiert, um farbige Verunreinigungen zu entfernen (Verbindung 1).

Man erhält 219,3 g (60,9% der Theorie) gelblichweisse Kristalle. Smp. 140–141°C.

Analyse: $C_{30}H_{29}N_3O$

| | | | |
|---|---|---|---|
| berechnet | C: 80,51 | H: 6,53 | N: 9,39 |
| gefunden | C: 80,53 | H: 6,54 | N: 9,51 |

Beispiel 2:

2-Nitro-2'-hydroxy-3',5'-di(α,α-dimethyl-benzyl)-azobenzol

In einem 500 ml Dreihals-Kolben mit Rührer, Gaseinleitung und Überdrucksicherung gibt man 13,5 g (0,21 Mol) festes Kaliumhydroxid und 10 ml Wasser. Die resultierende heisse Lösung wird mit 80 ml Methanol abgekühlt.

16,5 g (0,05 Mol) 2,4-Di-(α,α-dimethyl-benzyl)-phenol und 85 ml Methanol werden unter Stickstoff zugefügt und auf −4°C abgekühlt. Eine gekühlte Lösung enthaltend 42,9 g (0,06 Mol) o-Nitrobenzoldiazoniumchlorid in konz. HCl wird während 15 Minuten unter Rühren zugefügt und die Temperatur auf −2°C bis 0°C gehalten. Sofort entsteht eine tiefrote Färbung, verursacht vom gebildeten Azofarbstoff-Phenoxid. Das Gemisch wird bei −1°C bis +1°C 10 Minuten lang weitergerührt, anschliessend mit 20 ml Eisessig langsam (während 2 Minuten) angesäuert und die Temperatur bei +1 bis +3°C gehalten. Die ziegelrote Suspension wird 15 Minuten lang weitergerührt, wobei eine Temperatursteigerung möglich ist, und anschliessend abfiltriert. Man wäscht den Filterrückstand mit einer Lösung aus 40 g Eis und 160 ml Methanol und zum Schluss mit 1800 ml Wasser.

Das hellrote Rohprodukt wird im Vakuum bei 30°C und 75 mm Hg während 16 Stunden getrocknet. Die Ausbeute beträgt 22,7 g, 82% der Theorie. Die spectrophotometrische Analyse zeigt 86,7% Reinheit auf. Smp: 135–140°C.

Das Rohprodukt wird aus heissem n-Butanol (5 ml pro Gramm) umkristallisiert. Smp: 147–148°C.

Beispiel 3:

2-Nitro-2'-hydroxy-3',5'-di-(α,α-dimethyl-benzyl)-azobenzol

Unter Verwendung des Verfahrens von Beispiel 2, werden 13,6 g (0,34 Mol) feste Natronlauge in 145 ml Methanol gelöst, 16,5 g (0,05 Mol) 2,4-Di-(α,α-dimethyl-benzyl)-phenol und 20 ml Methanol zugefügt.

Man kühlt die Lösung auf +2°C ab. Inzwischen wird eine o-Nitrobenzol-diazoniumchlorid-Lösung aus 8,3 g (0,06 Mol) o-Nitroanilin und 17,3 g conc. HCl sowie 6 ml Wasser und aus 4,3 g Natriumnitrit in 8 ml Wasser hergestellt. Man gibt die Diazonium-Lösung 2 Stunden lang bei +2°C zu der alkalischen Lösung. Das tiefrote Gemisch wird 30 Minuten lang bei +2°C gerührt, dann mit 20 ml Eisessig angesäuert und der hellrote Niederschlag durch Filtrieren isoliert. Man wäscht das Produkt mit 3×50 ml Methanol, dann mit 4×75 ml Wasser und anschliessend trocknet man bei 75°C mm Hg. Die Ausbeute beträgt 21,6 g (89% mit 97% Reinheit. Smp: 140–142°C.

Beispiele 4–16:

Alkalische Kupplung

Verfahren wird wie in Beispiel 2. Man verwendet jedoch anstelle von 2,4-Di-(α,α-dimethyl-benzyl)-phenol andere Phenole. Die Ausbeute variiert entsprechend den Substituenten am Phenol.

| Beispiel Nr. | Phenole | Ausbeute o-Nitro-azobenzol in % |
|---|---|---|
| 4 | 2,4-Di-tert-amyl | 66 |
| 5 | 2,4-Di-tert-octil | 70(50)[a] |
| 6 | 2,4-Di-tert-octyl | 47[b] |
| 7 | 2,4-Di-(α,α-Dimethyl-benzyl) | 82 |
| 8 | 2,4-Di-(α,α-Dimethyl-benzyl) | 89[c] |
| 9 | 2-(1-Phenyläthyl)-4-methyl | 84.3 |
| 10 | 4-Methyl | 56[d] |
| 11 | 2,4-Di-n-octyl | 56[e] |
| 12 | 2-Methyl-4-α,α-dimethyl-benzyl | 85* |
| 13 | 2-(α,α-Dimethyl-benzyl-4-methyl | 66[f] |
| 14 | 2-(α,α-Dimethyl-benzyl)-4-tert-butyl | 54 |
| 15 | 2-(α,α-Dimethyl-benzyl)-4-tert-octyl | 62 |
| 16 | 2-tert-Octyl-4-(α,α-dimethyl-benzyl) | 73 |
| | Kontrolle ohne Phenol | 0[g] |

a) Verfahren wurde wie im Beispiel 3 mit hoher Alkalikonzentration. Die Ausbeute beträgt nach der Reinigung 50%. $N_2$ Gasentwicklung fand statt. (= 48% der Theorie).

b) $N_2$ Entwicklung = 60% der Theorie.

c) Verfahren nach Beispiel 3.

d) $N_2$ Gasentwicklung = 29% der Theorie.

e) Die saure Kupplung ergab nur 16–19% Ausbeute.

f) $N_2$ Gasentwicklung = 33% der Theorie.

g) Wenn kein Phenol vorhanden, etwa 67% der theoretisch vorhandenen $N_2$ entwickelt sich aus der zersetzten Diazoniumlösung.

* Die Ausbeute beträgt 47%, wenn 4-Chloro-2-nitrobenzoldiazoniumchlorid mit diesem Phenol gekuppelt wird.

Beispiel 17:

2-Nitro-2'-hydroxy-3',5'-di-]α,α-dimethyl-benzyl)-azobenzol

Wenn man im Beispiel 2 anstelle von Methanol Äthanol verwendet, wird eine $N_2$-Entwicklung (50% der Theorie) beobachtet. Man erhält eine niedrige Ausbeute (50,5%) des oben genannten Produktes.

Zum gleichen Ergebnis führt, wenn man anstelle von Methanol Isopropanol verwendet.

Diese Daten zeigen, dass Methanol das geeigneteste Lösungsmittel für die alkalische Kupplung ist.

Beispiel 18:
4-Chloro-2-nitro-2'-hydroxy-3',5'-d-(α,α-dimethyl-benzyl)-azobenzol

Wenn man im Vergleichsbeispiel B anstelle von der Diazoniumlösung, hergestellt aus 2-Nitroanilin, eine Diazoniumlösung, hergestellt aus 4-Chlor-2-nitroanilin in äquivalenter Menge verwendet, erhält man 47,°% vom dunkelroten Produkt.

Beispiel 19:
5-Chloro-2-]2'-hydroxy-3',5'-di-(α,α-dimethyl-benzyl)-phenyl]-2H-benzotriazol

Wenn man in Beispiel 1 anstelle von 2-Nitro-2'-hydroxy-3',5'-di-(α,α-dimethyl-benzyl)-azobenzol 4-Chloro-2-nitro-2'-hydroxy-3',5'-di-(α,α-dimethyl-benzyl)-azobenzol verwendet, erhält man 70,0% vom Produkt. Die hellbraunen Kristalle schmelzen bei 160-161°C. (Verbindung Nr. 2).

Analyse: $C_{30}H_{28}C N_3O$:

| | | | |
|---|---|---|---|
| berechnet | C: 74.45; | H, 5.86; | N: 8.72 |
| gefunden | C: 74.53; | H, 6.11; | N: 8.72 |

Beispiel 20:
2-(2-Hydroxy-3-tert.-octyl-5-α,α-dimethyl-benzyl)-phenyl-2H-benzotriazol

Wenn man im Beispiel 1 anstelle des o-Nitroazobenzols aus Vergleichsbeispiel B eine äquivalente Menge 2-Nitro-2'-hydroxy-3'-tert.-octyl-5'-α,α-dimethyl-benzyl-azobenzol verwendet, erhält man das oben genannte Produkt.

Beispiel 21:
Verlustwiderstand des Benzotriazols

Eine Anzahl von 2-Aryl-2H-benzotriazolen werden thermalgravimetrisch analysiert, und zwar sowohl isothermisch bei 280°C, um die Zeit in Minuten zu ermitteln, bei welcher 10%, 50% und 100% des Stabilisators verloren geht, als auch durch eine Auszähl-Methode, bei der die Temperatur kontinuierlich um 10°C pro Minute erhöht wurde, bis ein Stabilisatorschwund von 10% bzw. 50% festgestellt wurde.

Die Testergebnisse sind in der Tabelle A zusammengefasst.

Die Ergebnisse zeigen das genaue Mass an Stabilisatorwiderstand während der Verarbeitung des Polymeres zu Folien, Filmen, Fasern etc. nicht auszuschwitzen oder zu verflüchtigen.

Tabelle A

Thermalgravimetrische Analysedaten

| Stabilisator* | isometrisch bei 280°C Zeit in Minuten bis zum Gewichtsverlust von | | | Temperatur in °C bei einer Temperatur-Steigerung von 10°C/Minute bis zum Gewichtsverlust von | |
|---|---|---|---|---|---|
| | 10% | 50% | 100% | 10% | 50% |
| TINUVIN P | 0.4 | 0.75 | 1.2 | 182 | 215 |
| TINUVIN 350 | 0.6 | 1.0 | 1.8 | 210 | 247 |
| CYASORB UV-5411 | 0.6 | 1.9 | 3.5 | 225 | 260 |
| Verbindung 333 | 0.8 | 3.0 | 6.0 | 250 | 290 |
| Verbindung 1 | 6.0 | 24.0 | 56.0 | 300 | 340 |
| Verbindung 2 | 6.0 | 29.0 | 65.0 | 305 | 350 |
| Verbindung 3 | 1.7 | 6.3 | 12.5 | 273 | 313 |
| Verbindung S1 | 0.8 | 2.6 | 4.5 | 240 | 282 |
| Verbindung S2 | 1.0 | 4.0 | 7.0 | 257 | 298 |
| Verbindung S3 | 1.0 | 3.4 | 6.6 | 258 | 298 |
| Verbindung S4 | 0.8 | 2.8 | 5.7 | 263 | 298 |

* TINUVIN P ist 2-(2-Hydroxy-5-methylphenyl)-2H-benzotriazol. (GB 878.362)
* TINUVIN 350 ist 2-(2-Hydroxy-3-tert-butyl-5-sec-butylphenyl)-2H-benzotriazol. (DE-AS 2.130.322)

CYASORB UV-5411 ist 2-(2-Hydroxy-5-tert-octylphenyl)-2H-benzotriazol. (NL 127.157)

Verbindung 333 ist 2-([2-Hydroxy-3-(1-phenyläther)-5-methylphenyl]-2H-benzotriazol. (DE-AS 2.130.322)

Verbindung S1 ist 2-[2-Hydroxy-3-methyl-5-(α,α-dimethyl-benzyl)-phenyl]-2H-benzotriazol. (JP-OS 158.588/75)

Verbindung S2 ist 2-[2-Hydroxy-3-(α,α-dimethyl-benzyl)-5-methyl-phenyl]-2H-benzotriazol. (JP-OS 158.588/75)

Verbindung S3 ist 2-[2-Hydroxy-3-(α,α-dimethyl-benzyl)-5-tert-butyl-phenyl]-2H-benzotriazol. (JP-OS 158.588/75)

Verbindung S4 ist 5-Chloro-2-[2-hydroxy-3-methyl-5-(α,α-dimethyl-benzyl)-phenyl]-2H-benzotriazol. (JP-OS 158.588/75)

Die Verbindung 1 (aus Beispiel 1) ist eindeutig widerstandsfähiger als die anderen Benzotriazole. Die Verbindung 1, eingearbeitet in das Polymer, würde daher dem Polymer einen besseren Schutz gegen schädliche Lichteinwirkungen bieten und darüber hinaus ein ausgezeichnetes Verhalten während der Verarbeitung zeigen. Dasselbe gilt für die Verbindungen 2 (Beispiel 19) und 3 (Beispiel 28).

Beispiel 22:
Beständigkeit von Benzotriazol-Stabilisatoren in Polycarbonat während der Folienherstellung.

Polycarbonatharz, (Lexan, General Electric) wurde mit 0,3% (Gewicht) diverser 2-Aryl-2H-benzotriazol UV-Absorber ausgerüstet. Das formulierte Harz wurde extrudiert bei 316°C zu dünnen Folien. Die erhaltenen Folien wurden in Methylenchlorid gelöst und das Polycarbonat mit Methanol ausgefällt. Die Menge an Benzotriazol-Stabilisator, die in der Polycarbonat-Folie nach der Herstellung verblieb, wurde mittels Gaschromatographie-Analyse bestimmt. Die Ergebnisse zeigt Tabelle B.

Tabelle B

| Stabilisator* | % verblieben in der Polycarbonat-Folie nach Herstellung |
|---|---|
| TINUVIN 350 | 82 |
| CYASORB UV-5411 | 87 |
| Verbindung 333 | 100 |
| Verbindung 1 | 100 |

* siehe Tabelle A für die chemischen Namen dieser Stabilisatoren.

Diese Daten bestätigen die Ergebnisse von Tabelle A und die Sublimationsbeständigkeit und Exudationsbeständigkeit der Verbindung 1 während der Herstellung. Verbindung 33 zeigt in diesem Test ebenfalls eine geringe Flüchtigkeit.

Beispiel 23:
Beständigkeit gegen Verluste beim Härten und Bewittern von Benzotriazol-Stabilisatoren in Thermoset-Acrylüberzügen.

Diverse Thermoset-Acrylharze und ein Alkyd/-acrylharz-System wurden mit 2% (Gewicht) verschiedener Benzotriazol-UV-Absorber formuliert und auf Glasplatten zu 1µ dicken Überzügen gegossen. Die Überzüge wurden dann bei erhöhter Temperatur eine bestimmte Zeit gehärtet. Der Verlust an Benzotriazol-UV-Absorber wurde dann durch UV-Absorptions-Analyse der Überzüge bestimmt. Eine Abnahme der Absorbtion der Überzüge kann einen Verlust an Benzotriazol-Stabilisator während des Härtens gleichgesetzt werden.

Diese gehärteten Überzüge wurden dem beschleunigten (quick) Bewitterungstest (QUV) unterworfen, bei dem alternierend 4-Stunden UV-Bestrahlung bei 60°C und 4-Stunden Kondensation (Regen) bei 50°C über eine Zeitspanne von 670 Stunden einwirken. Wiederum kann eine Absorptionsabnahme der bewitterten Überzüge mit einem Verlust an Benzotriazol-Stabilisator während des Härtens und der Bewitterung gleichgesetzt werden. Die Ergebnisse zeigt Tabelle C.

Tabelle C

| | Absorptionsverlust während Härten oder Bewitterung (%) | | | | | | |
|---|---|---|---|---|---|---|---|
| Stabilisator* | Thermoset-Acrylharz-Systeme | | | | | Alkyd/Acrylharz | |
| | Einfache Schicht | | 2-Schicht-System | | High-Solids | | |
| | erhitzt 25 Min. bei 120°C | nach Bewitterung | erhitzt 20 Min. bei 135°C | nach Bewitterung | erhitzt 30 Min. bei 150°C | erhitzt 30 Min. bei 125°C | Nach Bewitterung |
| TINUVIN 328 | 25 | 35 | 59 | 69 | 95 | 77 | 100 |
| Verbindung 333 | 2 | 17 | 8 | 26 | 37 | 12 | 96 |
| Verbindung I | 0 | 5 | 0 | 10 | 0 | 0 | 73 |

*TINUVIN 328 ist 2-(2-Hydroxy-3,5-di-tert-amylphenyl)-2H-benzotriazol.
Verbindung 333 ist 2-[2-Hydroxy-3-(1-phenyläthyl)-5-methylphenyl]-2H-benzotriazol.

Tabelle C zeigt, dass die erfindungsgemässe Verbindung viel weniger Verluste in den thermoset Acryl-Harz- und Alkyd/Acrylharz-Systemen erleidet als bekannte Benzotriazole. Verbindung 1 ist beträchtlich weniger flüchtig als Verbindung 333, die eine vergleichbare Struktur hat.

Beispiel 24:
Glanzwerte von Acryl-Emaille und Lacken enthaltend Benzotriazol-Stabilisatoren und Bewitterung.

Diverse thermoset Acryl-Emaillen und thermoplastische Acryllacke wurden mit einem Benzotriazol-UV-Absorber formuliert. Die Glanzwerte werden mit der ursprünglichen Emaille oder dem Lack verglichen, nach beschleunigter Bewitterung (QUV mit je einem Zyklus 4 Stunden UV bei 60°C und 4 Stunden Kondensation bei 50°C). Die Ergebnisse zeigt Tabelle D.

Tabelle D

| | 20° Glanzwerte von Emaillen oder Lacken enthaltend Benzotriazol-Stabilisatoren vor und nach Bewitterung | | | | | |
|---|---|---|---|---|---|---|
| Stabilisator* | Thermoset Acryl-Emaille A | | Thermoset Acryl-Emaille B | | Thermoplastischer Acryl-Lack | |
| | vorher | 800 Stunden QUV | vorher | 800 Stunden QUV | vorher | 200 Stunden QUV |
| keine Stabilisatoren | 65 | 15.4 | 67.3 | 25.1 | 77.8 | 23.1 |
| 1% TINUVIN 328 | 68.1 | 15.0 | 64.6 | 26.7 | — | — |
| 1% Verbindung 333 | 68.7 | 19.8 | 65.2 | 25.6 | 78.0 | 24.0 |
| 1% Verbindung 1 | 66.9 | 27.8 | 68.8 | 33.2 | 78.5 | 41.1 |
| 2% TINUVIN 328 | 64.8 | 16.6 | 59.2 | 23.1 | 77.1 | 33.5 |
| 2% Verbindung 333 | 68.6 | 19.4 | 63.5 | 30.3 | 77.4 | 41.0 |
| 2% Verbindung 1 | 66.7 | 27.2 | 69.3 | 39.0 | 79.1 | 41.0 |

* Siehe Tabelle C für die chemischen Namen dieser Stabilisatoren.

Formulierungen mit Verbindung 1 ergaben durchgehend besseren Glanz nach QUV-Bewitterung als solche mit bisher bekannten Benzotriazolen.

Beispiel 25:
Stabilisation von Polyäthylen-terephthalat.

0,5% der Verbindung von Beispiel 1 wird als Stabilisator zu geschmolzenen Polyäthylen-terephthalat bei 270°C unter Rühren und einer Stickstoffatmosphäre gegeben. Der erhaltene, formulierte Polymer wird mit festem Kohlendioxid gemahlen.

Die stabilisierte Mischung wird bei erhöhter Temperatur zu einem Film unter geringem Stabilisatorverlust extrudiert. Dann wird der Film einer UV-Bestrahlung unterworfen. Der stabilisierte Film behält seine erwünschten physikalischen Eigenschaften länger als der unstabilisierte.

Beispiel 26:
Stabilisation von Polycarbonat.

Polycarbonat (Lexan, General Electric) wird in einem Extruder mit 0,3% der Verbindung von Beispiel 4 gemischt. Die stabilisierte Mischung wird extrudiert zu einer Folie bei erhöhter Temperatur unter geringem Verlust an Stabilisator. Die Folie behält ihre physikalischen Eigenschaften nach UV-Bestrahlung länger als eine solche ohne Stabilisator.

Beispiel 27:
2-Nitro-2'-hydroxy-3'-α,α-dimethylbenzyl-5-tert-octylazobenzol.

Diese Verbindung wird nach dem allgemeinen Verfahren des Beispiels 3 erhalten durch Kupplung von o-Nitrobenzol-diazonium-Chlorid mit 2-α,-Dimethylbenzyl-4-tert-octyl-phenol, als roter kristalliner Feststoff, F 133-134°C.

Beispiel 28:
2-(2-Hydroxy-3-α,α-dimethylbenzyl-5-tert-octylphenyl)-2H-benzotriazol.

Gemäss dem allgemeinen Verfahren von Beispiel 1 wird eine äquivalente Menge 2-Nitro-2'-hydroxy-3'-α,α-dimethylbenzyl-5'-tert-octylazobenzol anstelle des o-Nitroazobenzol-Zwischenproduktes des Vergleichsbeispiels B verwendet. Obige Verbindung wurde erhalten als fast weisse Kristalle, F. 88-90°C (Verbindung 3).

Analyse: Berechnet für $C_{29}H_{35}N_3O$:
C: 78,87; H, 7,99; N, 9,51
gefunden: C: 79,21; H, 8,01; N, 9,55.

Beispiel 29:
2-(2-Hydroxy-3-tert-octyl-5-α,α-dimethyl-benzylphenyl)-2H-benzotriazol.

Gemäss dem Verfahren des Beispiels 1 wird eine äquivalente Menge 2-Nitro-2'-hydroxy-3'-tert-octyl-5-α,α-dimethylbenzyl-azobenzol anstelle des o-Nitroazobenzol-Zwischenproduktes des Vergleichsbeispiels B verwendet. Man erhält obige Verbindung.

Die Delamination von UV-transparenten Automobil-Decklacken, aufgetragen auf Epoxy-Ester-Primer Oberflächen ist ein schwerwiegendes Problem der Automobilhersteller. Dieses Problem wird besonders relevant, wenn die Decklack-Stärke unter der Vorschrift liegt. Einarbeiten von erfindungsgemässen UV-Absorbern in den Decklack schützt diesen vor Delamination und vor Glanzverlust.

Beispiel 30:
Glanz- und Delaminations-Werte von Decklacken aus thermoplastischem Acrylat.

Ein silbermetall-thermoplastischer Acryllack wurde formuliert mit einem Benzotriazol-UV-Absorber und dann als Decklack auf eine Primer-Oberfläche aus einem Epoxy-Ester auf Metallatten gesprüht. Das Ganze wurde 10 Minuten bei

48°C und dann 30 Minuten bei 155°C erhitzt. Die ursprüngliche Decklackschichtdicke war 2,0 b 2,2 mils (50 bis 55 Micron, 0,0508 bis 0,0559 mm).

Die Platten wurden ein Jahr in Süd-Florida in einem ungeheizten schwarzen Kasten bei einem Winkel von 5° zur Sonne belichtet.

Die belichteten Platten wurden 96 Stunden in eine Kammer konstanter Feuchtigkeit bei 38°C und 100% relativer Feuchte gegeben. Dann wurden sie abgetrocknet und sofort begutachtet mittels des «cross-hatch tape» Adhäsionstests. Dann wurden die Platten eine Stunde bei Raumtemperatur belassen, bevor der «cross-hatch tape» Adhäsionstest an einer anderen Stelle derselben Platte wiederholt wurde. Die Proben zeigen allgemein eine etwas verbesserte Delaminationsbeständigkeit nach dieser einstündigen Erholungspause, gegenüber der Delamination sofort nach der Feuchtigkeitsbehandlung.

Beim «cross-hatch tape» Adhäsionstest wird ein Mehrschneidemesser benutzt, um Schnitte durch den Decklack auf der Platte anzubringen. Ein Acetatfaser-Klebeband wird über die Schnittstellen geklebt und dann abgezogen. Der Decklack wird visuell beurteilt, ob und wieviel abgezogen wurde. Dies ergibt eine relative Beurteilung der Delamination von 0 (keine «cross-hatch» Delamination) bis 5 (vollständige «cross-hatch» Delamination). Die Ergebnisse zeigen Tabellen E und F.

Tabelle E

| | 20° Glanzwerte von Silber-Metallic thermoplastischem Acryl Lack* nach 5° Süd-Florida Black Box Belichtung | | | |
|---|---|---|---|---|
| Stabilisatoren (% Gewicht)** | vorher | nach 6 Monaten (Florida) | nach 1 Jahr (Florida) | nach 1 Jahr Poliert*** |
| keiner | 77.8 | 15.1 | 1.6 | 4.4 |
| 1% Verbindung 333 | 78.0 | 33.5 | 3.7 | 6.6 |
| 1% Verbindung 1 | 78.5 | 44.0 | 13.4 | 29.5 |
| 2% TINUVIN 328 | 77.1 | 34.9 | 8.5 | 13.7 |
| 2% Verbindung 333 | 77.4 | 41.0 | 9.5 | 28.0 |
| 2% TINUVIN 350 | 77.5 | 37.5 | 6.0 | 24.3 |
| 2% Verbindung 1 | 79.1 | 53.4 | 15.8 | 28.9 |
| 2% Verbindung 2 | 79.4 | 50.0 | 17.7 | 38.4 |

*Thermoplastischer Acryllack aus einem Binder von 60% Poly(methyl)methacrylat, 20% Celluloseacetatbutyrat und 20% Weichmacher, etwa 3% metallischem Pigment.
** Siehe Tabellen A und C für chemische Namen dieser Stabilisatoren.
*** Eine Hälfte jeder Platte wurde mit DuPont Nr. 7 Autopoliermittel poliert.

Tabelle F

| | Delaminationswerte für Silber-Metallic thermoplastischen Acryllack* nach einem Jahr 5° Süd-Florida Black Box Belichtung | |
|---|---|---|
| | Delaminationswert (0 bis 5) | |
| Stabilisator** (% Gewicht) | Sofort nach Feuchtigkeitsbehandlung | Nach einer Stunde Erholung bei Raumtemperatur nach Feuchtigkeitsbehandlung |
| keiner | 5 | 5 |
| 1% Verbindung 1 | 2 | 1 |
| 2% TINUVIN 328 | 5 | 2 |
| 2% TINUVIN 350 | 3 | 2 |
| 2% Verbindung 333 | 5 | 4 |
| 2% Verbindung 1 | 2 | 2 |
| 2% Verbindung 2 | 4 | 3 |

* Siehe Beschreibung des Lacks zu Tabelle E.
** Siehe Tabelle A und C für chemische Namen dieser Stabilisatoren.

Die Daten der Tabelle E bestätigen, dass die Verbindung 1 und die des Beispiels 10 ausgezeichneten Schutz gegen Verluste während längerer Belichtung im Süd-Florida Black Box Test bewirken, verglichen mit anderen Benzotriazollichtschutzmitteln.

Die Daten der Tabelle F zeigen, dass die erfindungsgemässen Verbindungen, insbesondere Verbindung 1, einen thermoplastischen Acryl-Decklack vor Delamination nach 1 Jahr Süd-Florida Black Box Belichtung unter strengsten Testbedingungen mit sofort nachfolgender Feuchtigkeitsbehandlung schützen.

Beispiel 31:
Delaminationswerte von Decklacken aus thermoset, Acryl-Emaille.

Zwei silber-metallic thermoset Acryl-Emailles wurden mit einem Benzotriazol-Lichtschutzmittel formuliert und dann als Decklack auf eine Pirmeroberfläche aus einem Epoxy-Ester auf einer Metallplatte gesprüht. 17 Minuten lang wurde bei 130°C gehärtet zu einem Anfangsdecklackfilm von 1,7 mil Dicke (42 Micron, 0,0432 mm). Die Platte wurde 1200 Stunden mit dem QUV Bewitterungstest gemäss Beispiel 23 behandelt.

Nach der QUV-Belichtung wurden die Platten feuchtigkeitbehandelt und dann die Delamination bestimmt, dann eine Stunde Erholung eingeschaltet, worauf erneut die Delaminationsbeständigkeit gemäss Beispiel 30 bestimmt. Die Ergebnisse zeigt Tabelle G.

Tabelle G

| | Delaminationswerte einer Silber-Metallic thermoset Acryl-Emaille nach 1200 Stunden QUV Belichtung | | | |
|---|---|---|---|---|
| | Delaminationswert (0 bis 5) Emaille* A | | Emaille* B | |
| Stabilisator (% Gewicht) | Sofort nach Feuchtigkeits-Behandlung | Nach 1 Std. Erholung bei Zimmertemperatur nach Feuchtigkeits-behandlung | Sofort nach Feuchtigkeits-behandlung | Nach 1 Std. Erholung bei Zimmertemperatur nach Feuchtigkeitsbehandlung |
| keiner | 3 | 1 | 5 | 3 |
| 1% Verbindung 1 | 0 | 0 | 0 | 0 |
| 2% Verbindung 1 | 0 | 0 | 0 | 0 |

* Thermoset Acryl-Emaille aus einem Binder von 70% Acrylmonomeren wie Hydroxyäthyl-acrylat, Styrol, Acrylnitril, Butyl-acrylat und Acrylsäure mit 30% eines Melaminharzes. Emaille A ist eine Nieder(40-45%)-Fertig-Lösung. Emaille B ist eine nicht-wässrige Dispersion (NAD) mit etwa 40-50% Feststoff und etwa 3% metallischem Pigment.

Die Kombination von sterisch gehinderten Amin-Lichtschutzmitteln mit den erfindungsgemässen Benztriazol-UV-Absorbern ist besonders geeignet, den Glanz zu erhalten und Delamination zu verhindern, insbesondere bei metallisierten thermoset Acryl-Emaillen und bei metallisierten thermoplastischen Acryllacken für Automobildecklacken.

Die gehinderten Amine schützen die termoset Acryl-Emaille und den thermoplastischen Acryllack selbst bei niedriger Konzentration gegen Glanzverluste, wirken aber nicht als UV-Filter. Demgemäss kann UV-Licht durch den Acryldecklack treten, falls ein UV-Absorber abwesend ist, wie die erfindungsgemässen Benztriazole, und kann Schaden anrichten in der Epoxy-Esterprimeroberfläche unter dem Decklack. Einarbeiten von selbst niedrigen Konzentrationen (0.5% Gewicht) eines Benztriazols in Kombination mit einem gehinderten Amin bewirkt sowohl Glanzerhaltung als auch Widerstandsfähigkeit gegen Delamination für metallisierte Acryl-Decklacke.

Beispiel 32:
Delaminationswerte für Decklacke aus thermoset Acryl-Emaille.

Zwei silber-metallisierte thermoset Acryl-Emaillen wurden formuliert sowohl mit einem gehinderten Amin Lichtschutzmittel als auch einem Benztriazol-UV-Absorber. Die Testplatten wurden hergestellt und getestet wie im Beispiel 31 beschrieben. Die Ergebnisse zeigt Tabelle H.

Tabelle H

| | Delaminationswerte einer Silber-Metallic thermoset Acryl-Emaille nach 1200 Stunden QUV Belichtung | | | |
|---|---|---|---|---|
| | Delaminationswerte (0 bis 5) Emaille A | | Emaille B | |
| Stabilisator** (% Gewicht) | Sofort nach Feuchtigkeits-Behandlung | Nach 1 Std. Erholung bei Zimmertemperatur nach Feuchtigkeits-behandlung | Sofort nach Feuchtigkeits-behandlung | Nach 1 Std. Erholung bei Zimmertemperatur nach Feuchtigkeitsbehandlung |
| keiner | 3 | 1 | 5 | 3 |
| 0.5% Verbindung A | 1 | 0 | 5 | 2 |
| 1% Verbindung A | 3 | 0 | 5 | 1 |
| 2% Verbindung A | 3 | 0 | 5 | 1 |
| 0.5% Verbindung B | 5 | 2 | 5 | 3 |
| 1% Verbindung B | 4 | 2 | 5 | 0 |
| 2% Verbindung B | 2 | 2 | 5 | 0 |
| 1% TINUVIN 328 | 0 | 0 | 0 | 0 |
| 2% TINUVIN 328 | 0 | 0 | 0 | 0 |
| 1% Verbindung 1 | 0 | 0 | 0 | 0 |
| 2% Verbindung 1 | 0 | 0 | 0 | 0 |
| 1% Verbindung A + 1% TINUVIN 328 | 0 | 0 | 0 | 0 |
| 1% Verbindung B+ 1% TINUVIN 328 | 0 | 1 | 0 | 0 |
| 1% Verbindung A+ 1% Verbindung 333 | 0 | 0 | 0 | 0 |
| 1% Verbindung B+ 1% Verbindung 333 | 1 | 0 | 0 | 0 |
| 1% Verbindung A+ 1% Verbindung 1 | 0 | 0 | 0 | 0 |
| 1% Verbindung B+ 1% Verbindung 1 | 0 | 0 | 0 | 0 |

* Siehe Beschreibung der thermoset Acryl-Emaille in Tabelle G.
** Siehe Tabelle C für chemische Namen von Tinuvin 328 und Verbindung 333.

Verbindung A ist Bis-(2,2,6,6-tetramethyl-4-piperidyl)-sebacat.

Verbindung B ist Bis-(1,2,2,6-pentamethyl-4-piperidyl)-2-n-butyl-2-(3,5-di-tert-butyl-4-hydroxybenzyl)-malonat.

Beispiel 33:
Delamination und 20° Glanzwerte von thermoplastischen Acryllacken.

Die Wirksamkeit von Kombinationen von gehinderten Amin-Lichtschutzmitteln mit erfindungsgemässen Benztriazol-UV-Absorbern zum Schutz von Automobildecklacken zeigt sich bei thermoplastischen Acrylharzen, wobei sowohl Glanzerhalt nach längerer Belichtung in Süd-Florida durch das gehinderte Amin erfolgt, als auch durch die erfindungsgemässen Bentriazol-UV-Absorber Schutz des thermoplastischen Acryl-Decklackes vor Delamination.

Ein silber-metallic thermoplastischer Acryllack wurde formuliert sowohl mit einem gehinderten Amin-Lichtschutzmittel, als auch einem Benztriazol-UV-Absorber. Testplatten wurden hergestellt und getestet wie in Beispiel 30 beschrieben. Die Ergebnisse zeigt Tabelle J.

Tabelle J

| | Delamination und 20° Glanzwerte eines Silber Metallic thermoplastischen Acryllacks* nach 5° Süd-Florida Black Box Belichtung | | | | | |
|---|---|---|---|---|---|---|
| | 20° Glanzwerte | | | | Delaminationswerte (0 bis 5) nach 1 Jahr Belichtung | |
| Stabilisator** (% Gewicht) | vorher | nach 6 Monaten Florida | nach 1 Jahr Florida | nach 1 Jahr Florida poliert | sofort nach Feuchtigkeitsbehandlung | nach 1 Stunde Erholung bei Raumtemperatur nach Feuchtigkeitsbehandlung |
| keiner | 77.8 | 15.1 | 1.6 | 4.4 | 5 | 5 |
| 0.5% Verb.A | 77.5 | 48.0 | 15.1 | 35.6 | 5 | 3 |
| 1% Verb. A | 77.2 | 42.0 | 36.2 | 39.7 | 5 | 5 |
| 2% Verb. A | 76.7 | 44.2 | 14.3 | 31.4 | 5 | 5 |
| 0.5% Verb. B | 77.9 | 45.0 | 38.5 | 45.6 | 5 | 5 |
| 1% Verb. B | 77.4 | 45.4 | 22.1 | 37.7 | 5 | 2 |
| 2% Verb. B | 76.8 | 45.3 | 26.5 | 46.0 | 5 | 3 |
| 2% TINUVIN 328 | 77.1 | 34.9 | 8.5 | 13.7 | 5 | 2 |
| 2% DOBP | 77.5 | 35.0 | 6.8 | 19.7 | 3 | 2 |
| 1% Verb. 333 | 78.0 | 33.5 | 3.7 | 6.6 | — | — |
| 2% Verb. 333 | 77.4 | 41.0 | 9.5 | 28.0 | 5 | 4 |
| 1% Verb. 1 | 78.5 | 44.0 | 13.4 | 29.5 | 2 | 1 |
| 2% Verb. 1 | 79.1 | 53.4 | 15.8 | 28.9 | 2 | 2 |
| 1% Verb. 2 | 79.4 | 50.0 | 17.7 | 38.4 | 4 | 3 |
| 2% TINUVIN 350 | 77.5 | 37.5 | 6.0 | 24.3 | 3 | 2 |
| 1% Verb. A+ 1% TINUVIN 320 | 77.6 | 47.8 | 19.6 | 41.3 | 5 | 5 |
| 0.5% Verb. A+ 0.5% Verb. 333 | 78.3 | 50.2 | 21.4 | 39.4 | 5 | 4 |
| 0.5% Verb. B+ 0.5% Verb. 333 | 78.2 | 52.7 | 18.8 | 41.3 | 3 | 2 |
| 1% Verb. A+ 1% Verb. 333 | 78.4 | 49.6 | 21.7 | 39.0 | 5 | 4 |
| 1% Verb. B+ 1% Verb. 333 | 77.7 | 47.5 | 25.4 | 44.6 | 4 | 3 |
| 0.5% Verb. A+ 0.5% Verb. 1 | 78.2 | 47.1 | 16.0 | 37.2 | 3 | 2 |
| 0.5% Verb. B+ 0.5% Verb. 1 | 77.7 | 48.0 | 33.4 | 39.8 | 3 | 2 |

* Siehe Beschreibung des thermoplastischen Acryllacks in Tabelle E.
** Siehe Tabellen A, C und H für chemische Namen der Stabilisatoren. DOBP ist 4-Dodecyloxy-2-hydroxybenzophenon.

## Patentansprüche

1. Verbindungen der Formel I

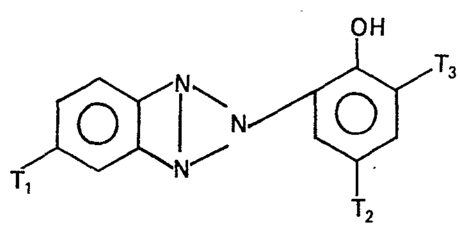

I

worin $T_1$ Wasserstoff oder Chlor ist, $T_2$ und $T_3$ unabhängig voneinander die Gruppe

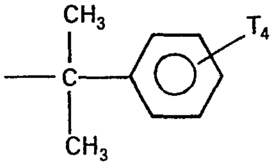

bedeuten, worin $T_4$ Wasserstoff oder niedriges Alkyl ist, wobei eines von $T_2$ und $T_3$ auch t-Octyl sein kann.

2. Verbindungen gemäss Anspruch 1, worin $T_1$ Wasserstoff oder Chlor ist, $T_2$ und $T_3$ die gleiche Bedeutung haben und $T_4$ Wasserstoff oder p-Methyl ist.

3. Verbindungen gemäss Anspruch 1, worin $T_4$ Wasserstoff ist.

4. Verbindung gemäss Anspruch 1, dadurch gekennzeichnet, dass es 2-[2-Hydroxy-3,5-di-(α,α-dimethylbenzyl)-phenyl]-2H-benzotriazol ist.

5. Verbindung gemäss Anspruch 1, dadurch gekennzeichnet, dass es 5-Chloro-2-[2-hydroxy-3,5-di-(α,α-dimethylbenzyl)-phenyl]-2H-benzotriazol ist.

6. Stabilisiertes organisches Material enthaltend 0,1-5 Gew.-% eines Stabilisators gemäss Anspruch 1-5.

7. Stabilisiertes organisches Material gemäss Anspruch 6, dadurch gekennzeichnet, dass das Material ein synthetisches Polymer ist.

8. Stabilisiertes Polymer gemäss Anspruch 7 und 6, dadurch gekennzeichnet, dass das Polymer ein Polyester, Polycarbonat, heissvernetzbares Acrylharz, Polyamid oder ein Polyurethan ist.

9. Stabilisiertes Polymer gemäss Anspruch 8 in Form eines Lacks mit Glanzhaltung und Abblätterungsbeständigkeit bei Bewitterung, gekennzeichnet durch

a) heissvernetzbares Acrylharz und

b) 0,1 bis 5 Gew.-%, bezogen auf das Harz, einer Verbindung der Formel I gemäss Anspruch 1-5, und

c) 0,1 bis 5 Gew.-%, bezogen auf das Harz, eines sterisch gehinderten Amin-Stabilisators.

10. Stabilisiertes organisches Material gemäss Anspruch 9, enthaltend 0,5 bis 2 Gew.-% von b) und 0,5 bis 2 Gew.-% von c).

11. Stabilisiertes organisches Material gemäss Anspruch 9, enthaltend als Bestandteil c) Bis-(2,2,6,6-tetramethyl-4-piperidyl)-sebacat als Lichtschutzmittel.

12. Stabilisiertes organisches Material gemäss Anspruch 9, enthaltend als Bestandteil c) Bis-(1,2,2,6,6-pentamethyl-4-piperidyl)-2-n-butyl-2-(3,5-di-tert.-butyl-4-hydroxybenzyl)-malonat als Lichtschutzmittel.

**Claims**

1. A compound of the formula

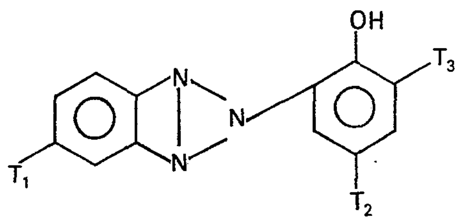

I

wherein $T_1$ is hydrogen or chlorine and each of $T_2$ and $T_3$ independently of the other is the group

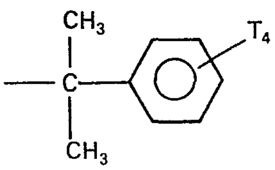

wherein $T_4$ is hydrogen or lower alkyl, whilst one of $T_2$ and $T_3$ can also be t-octyl.

2. A compound according to claim 1, wherein $T_1$ is hydrogen or chlorine, $T_2$ and $T_3$ have the same meaning and $T_4$ is hydrogen or p-methyl.

3. A compound according to claim 1, wherein $T_4$ is hydrogen.

4. 2-[2-Hydroxy-3,5-di-(α,α-dimethylbenzyl)-phenyl]-2H-benzotriazole, according to claim 1.

5. 5-Chloro-2-[2-hydroxy-3,5-di-(α,α-dimethylbenzyl)-phenyl]-2H-benzotriazole according to claim 1.

6. A stabilised organic material containing 0,1-5% by weight of a stabiliser according to any one of claims 1 to 5.

7. A stabilised organic material according to claim 6, wherein the material is a synthetic polymer.

8. A stabilised polymer according to claim 6, wherein the polymer is a polyester, polycarbonate, hot-crosslinkable acrylic resin or polyamide or a polyurethane.

9. A lacquer which has gloss retention and resistance to peeling on weathering, said lacquer containing

a) a hot-crosslinkable acrylic resin and

b) 0,1 to 5% by weight, based on the resin, of a compound of the formula I according to any one of claims 1 to 5 and

c) 0,1 to 5% by weight, based on the resin, of a sterically hindered amine stabiliser.

10. A stabilised organic material according to claim 9, containing 0,5 to 2% by weight of b) and 0,5 to 2% by weight of c).

11. A stabilised organic material according to claim 9, containing, as constituent c), bis-(2,2,6,6-tetramethyl-4-piperidyl) sebacate as light stabiliser.

12. A stabilised organic material according to claim 9, containing, as component c), bis-(1,2,2,6,6-pentamethyl-4-piperidyl)-2-n-butyl-2-(3,5-di-tert.-butyl-4-hydroxybenzyl)-malonate as light stabiliser.

**Revendications**

1. Composés répondant à la formule I

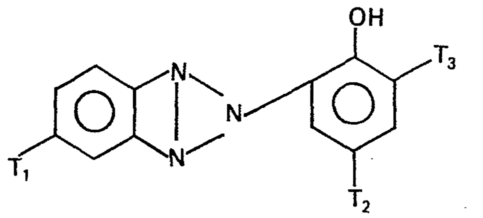

I

dans laquelle $T_1$ représente l'hydrogène ou le chlore, et $T_2$ et $T_3$ représentent chacun, indépendamment l'un de l'autre, un radical:

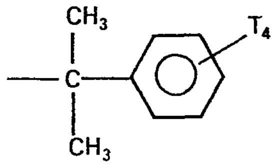

dans lequel $T_4$ représente l'hydrogène ou un radical alkyle inférieur, l'un des symboles $T_2$ et $T_3$ pouvant également être un radical tert-octyle.

2. Composés selon la revendication 1 dans lesquels $T_1$ représente l'hydrogène ou le chlore, $T_2$ et $T_3$ ont la même signification et $T_4$ représente l'hydrogène ou un radical méthyle en para.

3. Composés selon la revendication 1 dans lesquels $T_4$ représente l'hydrogène.

4. Composé selon la revendication 1, en l'espèce l'[hydroxy-2-bis-(α,α-diméthylbenzyl)-3,5-phényl]-2-2H-benzotriazole.

5. Composé selon la revendication 1, en l'espèce le chloro-5-[hydroxy-2-bis-(α,α-diméthylbenzyl)-3,5-phényl]-2-2H-benzotriazole.

6. Matière organique stabilisée qui contient de 0,1 à 5% en poids d'un stabilisant selon l'une quelconque des revendications 1 à 5.

7. Matière organique stabilisée selon la revendication 6, caractérisée en ce que la matière est un polymère synthétique.

8. Polymère stabilisé selon la revendication 6, caractérisé en ce que le polymère est un polyester, un polycarbonate, une résine acrylique réticulable à chaud, un polyamide ou un polyuréthanne.

9. Vernis conservant son brillant et résistant bien à l'écaillage lorsqu'il est exposé aux agents atmosphériques, vernis qui renferme:

a) une résine acrylique réticulable à chaud,

b) de 0,1 à 5% en poids, par rapport à la résine, d'un composé de formule I selon l'une quelconque des revendications 1 à 5 et

c) de 0,1 à 5% en poids, par rapport à la résine, d'un stabilisant qui est une amine à empêchement stérique.

10. Matière organique stabilisée selon la revendication 9, qui contient de 0,5 à 2% en poids de b) et de 0,5 à 2% en poids de c).

11. Matière organique stabilisée selon la revendication 9, qui renferme, comme constituant c), du sébaçate de bis-(tétraméthyl-2,2,6,6-pipéridyle-4) jouant le rôle de stabilisant à la lumière.

12. Matière organique stabilisée selon la revendication 9, qui renferme, comme constituant c), du n-butyl-2-(di-tert-butyl-3,5-hydroxy-4-benzyl)-2-malonate de bis-(pentaméthyl-1,2,2,6,6-pipéridyle-4) jouant un rôle de stabilisant à la lumière.